# EUROPEAN PATENT SPECIFICATION

(11) **EP 2 992 953 B1**
(45) Date of publication and mention of the grant of the patent: **26.05.2021**
(21) Application number: 14708924.7
(22) Date of filing: 23.01.2014
(51) Int. Cl.: B01J 13/16

(54) **METHOD FOR PRODUCING A MICROENCAPSULATE, MICROENCAPSULATE AND COMPOSITION.**
VERFAHREN ZUR HERSTELLUNG EINES MIKROKAPSULATS, MIKROKAPSULAT UND ZUSAMMENSETZUNG
PROCÉDÉ POUR LA FABRICATION D'UN MICRO-ENCAPSULÉ, MICRO-ENCAPSULÉ ET COMPOSITION CORRESPONDANTS

(30) Priority: 25.01.2013 ES 201330086
(43) Date of publication of application: 09.03.2016
(73) Proprietor: Ecopol Tech, S.L., 43720 L'Arboç (ES)
(72) Inventor: ROCAS SOROLLA, Josep, E-08860 Castelldefels (Barcelona) (ES); ROCAS ALONSO, Pau, E-08860 Castelldefels (Barcelona) (ES); MAS MORUNO, Carlos, E-08024 Barcelona (ES)
(74) Representative: Curell Suñol S.L.P.
(86) International application number: PCT/ES2014/070046
(87) International publication number: WO 2014/114838

(56) References cited:
- WO-A1-01/19509
- WO-A2-2008/021800
- WO-A2-2012/140302
- GB-A- 2 135 469
- US-A- 3 875 074
- US-A- 5 342 556
- US-A1- 2002 040 065
- US-A1- 2004 197 357
- US-A1- 2007 166 697
- US-A1- 2007 270 508
- DATABASE WPI Week 201257 29 September 2011 (2011-09-29) Thomson Scientific, London, GB; AN 2011-N72619 XP002726860, & CN 102 198 386 A (UNIV QINGDAO SCI&TECHNOLOGY) 28 September 2011 (2011-09-28)
- LIN J J ET AL: "Phase inversion of self-aggregating Mannich amines with poly(oxyethylene) segments", JOURNAL OF COLLOID AND INTERFACE SCIENCE, ACADEMIC PRESS, NEW YORK, NY, US, vol. 258, no. 1, 1 February 2003 (2003-02-01), pages 155-162, XP027136630, ISSN: 0021-9797 [retrieved on 2003-02-01]

## Description

### Field of the invention

The invention relates to a method for producing a microencapsulate comprising the following stages: [a] dispersing a first liquid phase in a second liquid phase forming an emulsion, so that the first phase is dispersed in the second phase, and [b] forming a polymer that forms the wall of the microencapsulate.

The invention also relates to a microencapsulate and a cosmetic or pharmaceutical composition comprising a microencapsulate.

### State of the art

Various encapsulation methods are known. However most of them require high stilling, high concentrations of external emulsifying agents or solvent to obtain capsule sizes smaller than one micron, etc.

It is known to use emulsifying agents which are, simultaneously, prepolymers of the polymer that will form part of the microencapsulate wall. Some examples can be found in documents US 6.262.152, US 7.199.185 and US 2007/0270508.

Document US 3.875.074 A describes a microencapsulation system, more particularly, a process for the encapsulation of minute oil droplets, the microcapsules produced thereby, and pressure-responsive, transfer-copy systems employing such microcapsules.

Document US 2004/197357 A1 describes reactive polymeric surfactants, and their use in processes for preparation of microcapsules, and in stabilising emulsions. The surfactants are random graft polymers or block copolymers which contain both hydrophobic and hydrophilic units and in which the hydrophobic unit includes a hydrophilic cross-linking unit which reacts with (a) a wall forming ingredient in a microencapsulation process, or (b) an ingredient in the disperse phase of an emulsion.

Document WO 2012/140302 A2 describes a method for manufacturing a microencapsulate of a hydrophobic active ingredient and a microencapsulate and corresponding compositions. The method for manufacturing a microencapsulate of a hydrophobic active ingredient, 90% of the particles of which are smaller than 5 micras, includes the following steps: [a] preparing a first solution of 5% or more of polyvinyl alcohol in water, [b] preparing a second solution of a hydrophobic active ingredient and 5% to 50% of a polyisocyanate, [c] preparing a third solution of a polyamine from the ethylendiamine and diethylentriamine group, [d] mixing the first solution with the second solution and agitating same to create an aqueous dispersion, and [e] mixing the aqueous dispersion from step [d] with the third solution, such that the proportion of the hydrophobic phase with respect to the aqueous phase is between 1 :1 and 1 :4, and allowing same to react.

Document US 5.342.556 A describes how microcapsules can be prepared in an energy-saving manner by using a polyisocyanate which consists exclusively or partially of reaction products of isocyanates having a functionality of 2 or more with polyethylene oxide alcohols.

Generally, emulsifying agents can be of two main types: oil in water (normally indicated as O/W) or water in oil (normally indicated as W/O). Generally this invention is suitable in both cases, although the most usual are the O/W emulsions and therefore most of the examples will be of this type, but at no time must the invention be understood to only refer to oil in water emulsions. In this specification and claims the term "first phase" has been used to indicate what will be the could have been classified instead as nanocapsules and/or nanoparticles.

### Disclosure of the invention

The aim of the invention is a new method which allows obtaining small size microcapsulates, and in relatively high concentrations. This purpose is obtained by means of a method according to the invention of the type indicated at the beginning characterised in that between the first phase and the second phase an interphase is formed that comprises a reactive amphiphilic compound, where the amphiphilic compound is a prepolymer of the polymer that will form the wall of the microencapsulate, and the amphiphilic compound has at least two main functional groups that react in the subsequent polymerisation to form the polymer that will form the wall of the microencapsulate, where the amphiphilic compound has at least one hydrophilic or hydrophobic functional group in a chain that is sideways with respect to the chain that joins both main functional groups, where the main functional groups of the amphiphilic compound are NCO functional groups, able to form urethane and/or urea type bonds, where the second phase comprises a second compound, where the second compound comprises at least two functional groups that react with the main functional groups of the amphiphilic compound, to form the polymer,
where the second compound is a polyamine, preferably a diamine, triamine, tetraamine or pentaamine and very preferably it is a polyamine from the group made up of ethylendiamine, diethylentriamine, triethylentetraamine and L-lysine,
where a second prepolymer is added to the first phase, where the second prepolymer has at least two main functional groups that react in the subsequent polymerisation for forming the polymer, where the second prepolymer is more hydrophobic than the amphiphilic compound if the first phase is organic, or the second prepolymer is more hydrophilic than the amphiphilic compound if the first phase is aqueous, so that the second prepolymer will tend to arrange itself as a second internal layer in the wall of the microcapsules, given its greater affinity to the first phase, and so that the second prepolymer will react also with the second compound thereby forming a wall with two layers, and
where the polymer comprises, in its main chain, a disulphide or an ester.

In fact the method according to the invention uses amphiphilic compounds that behave as dispersants and which self-emulsify the emulsion but which, at the same time, are reactive, which means that later they can be used to encapsulate, forming the wall of the microencapsulate. These amphiphilic compounds comprise a main chain, which preferably is "short", and they have at least one of the hydrophilic and hydrophobic functions in side chains. In fact this is a very interesting concept because it means amphiphilic compounds that are particularly suitable for the invention can be obtained. It must be taken into account that in this specification and claims the term "main chain" has been used to designate the one that has at the ends thereof the two functional groups (called main functional groups) which subsequently polymerise to form the polymer in the wall of the microencapsulate. This way, the amphiphilic compound is placed in the interphase between the two phases and is orientated so that it has the hydrophobic functions aimed towards the organic phase and the hydrophilic functions aimed towards the aqueous phase. This allows for a series of advantages. Between the two main functional groups there is little separation, this favours polymerisation, improves the barrier effect to prevent the diffusion of the encapsulated material towards the outside of the capsule, and makes encapsulation stable. For their part, the side chains are relatively flexible and adaptable.

Preferably, main "short" chain must be understood to mean that the two main functional groups of the amphiphilic compound are separated from each other by between 4 to 12 bonds, preferably between 5 to 10 bonds. Generally, the bonds will be of the -CH2- type, but they could be of another type (for example, substituents like O, N, S etc.). In fact, if there are more CH2 groups between the main functional groups, then the actual chain would already have a significant hydrophobic effect, which would counter the "side amphiphilic" concept and, also, it would have a negative effect on the rigidity (reducing it), as a greater number of CH2 groups in the main chain reduces the partial glass transition temperature (Tg). Therefore it is interesting to have a main short chain (with high rigidity and barrier effect) and to have longer side chains with the hydrophilic and hydrophobic functions.

So, for example in the case of O/W emulsions, the corresponding microcapsule will have an organic phase inside. The amphiphilic compound will have arranged itself so that its hydrophobic side chains are towards the hydrophobic inside of the capsules, and its hydrophilic side chains are towards the outside of the microcapsule and, during polymerising, this structure "froze" like this. This makes it possible later to obtain very stable microencapsulate dispersions, which do not aggregate, not even in the presence of saline or biological liquids that affect or promote their aggregation.

The prepolymer must have at least two main functional groups that react in the subsequent polymerisation for forming the polymer that will form the wall of the microencapsulate, however it can have more than two functional groups, with which better cross-linking will be achieved. Also it can have a mixture of amphiphilic compounds with a different number of main functional groups, so that the mixture makes it possible to obtain the degree of cross-linking required. In the event that the prepolymer has more than two main functional groups, it will be considered as the shortest main chain out of the possible chains that can be defined between the main functional groups.

Preferably the polymer (which forms the wall of the microencapsulate) is insoluble in the two phases, which is particularly important for obtaining a good barrier effect. In this respect, a particularly advantageous solution is that the polymer has urea and/or urethane type groups, and that these groups are near to one another.

As it can be seen, the invention includes the basic concept of generating a dispersion from a first phase and a second phase, where the interphase is formed thanks to an amphiphilic compound. The amphiphilic compound is a reactive compound. By virtue of this, after forming the dispersion, the amphiphilic compound reacts forming a polymer that forms the wall of the microcapsules. Therefore, the amphiphilic compound is really a prepolymer of the polymer that forms the wall of the microcapsules. This method allows very small (even nanometric size) microencapsulates to be formed and in relatively high concentrations. It must be taken into account that, generally, when microcapsules are prepared, if they are not well stabilised and diluted, they tend to agglomerate, and when they dry they tend to join together and the wall of the capsules tends to break because of the force of the actual agglomeration and their thin walls. The microcapsules according to the invention can be air-dried where they agglomerate relatively little at relatively high concentrations. Also, the dispersions are formed with moderate stirring, which means that active ingredients "sensitive" to stirring can be encapsulated and, at the same time, large-scale industrial manufacturing is possible with less energy and less expensive stirring equipment.

In the known microencapsulating methods, in the first phase a prepolymer is added which subsequently will form the polymer that shapes the wall of the microcapsules. For example, it is known to use polyisocyanates to produce microcapsules from an O/W solution. However, these prepolymers (like the polyisocyanates used normally) are not amphiphilic or self-emulsifying. For example, one polyisocyanate used is isophorone diisocyanate (IPDI). IPDI cannot be considered to be amphiphilic because it does not have one part soluble in water and one part soluble in oil. Also, IPDI does not migrate to the interphase, because it is soluble in oil and its solubility in water is not significant. Therefore, it is necessary to add, usually in the continuous phase (which is normally the aqueous phase) some dispersing and/or emulsifying agents external to the prepolymer. These dispersing and/or emulsifying agents, for their part, can be amphiphilic, but they are not reactive, and therefore they are not part of the polymer that shapes the wall of the capsules. However, these agents can hinder the wall formation process. Generally, in the known methods, there are more spaces between the polymer cross-links, it is harder to arrange themselves in the interphase and there are interferences between the reagents and the remaining agents in the interphase. In this invention the same amphiphilic compound that is the emulsifying agent and forms the emulsion is also the one that is reactive and is therefore, a prepolymer of the polymer that will form the wall of the microcapsules. This way, a wall with an improved barrier effect is obtained.

Based on this basic concept, the method according to the invention contemplates different variants. In fact:
- The amphiphilic compound can be added already-formed in the first phase, before forming the emulsion.
- The amphiphilic compound is formed in the first phase by reacting some of the precursors in the first phase, before forming the emulsion.
- The amphiphilic compound is formed "in situ" in the interphase of the emulsion, from precursors that are in the first phase and in the second phase, and that meet each other and react in the interphase.
- The polymer is formed by polymerising the amphiphilic compound with a second compound, added in the second phase, or added in the already-formed dispersion.

Also, there may be methods wherein several of the above alternatives converge.

If the amphiphilic compound is formed before making the emulsion, the amphiphilic compound is such that, being amphiphilic, it is less soluble in the first phase than in the second phase. This way, the amphiphilic compound tends to go to the interphase due to its affinity with the second phase. This way the amphiphilic compound acts as the emulsifying agent (self-emulsifying) and, also, it positions itself appropriately for the subsequent polymerisation reaction.

Preferably the amphiphilic compound, which is the prepolymer that will subsequently form the polymer that shapes the wall of the microencapsulate, is the main emulsifying agent in the emulsion and, very preferably, it is the only emulsifying agent. This aspect is particularly important because it avoids external emulsifying agents, which often can precipitate when they come into contact with the microcapsules in saline media or biological liquids (like blood or lymphatic liquid), and aggregate, which could cause damage to the biological organisms due to blocked arteries or veins, etc.

Advantageously the amphiphilic compound and/or the product derived from its polymerisation is the main stabilising agent, preferably it is the only stabilising agent in the emulsion.

Preferably no emulsifying and/or additional stabilising agent is added. In particular, the aqueous phase (particularly, when it is the second phase, i.e. the continuous phase) is free of emulsifying agents in the group made up alkyl carboxylates, alkyl sulphonates, alkyl ethoxylates (like the products sold under the names Tween ® and Span ®) and non-reactive amphiphilic polymers with the hydrophilic and hydrophobic chains in the main chain (like polypropylene glycol and polyethylene glycol, sold under the name Pluronic ®).

As already mentioned above, an alternative is that the first phase be the organic phase and the second phase be the aqueous phase. In this case it is advantageous that the amphiphilic compound has an HLB value (Hydrophilic lipophilic balance) higher than 10, preferably greater than 15. On the other hand, in the event that the first phase is the aqueous phase and the second phase is the organic phase, then it is advantageous that the amphiphilic compound has an HLB value less than 10. Generally, changing the proportion of lipophilic to hydrophilic functional groups in the prepolymer can obtain water in oil or oil in water type emulsions. Depending on the type of active ingredients that are to be encapsulated, one method or the other will be used. In the case of the oil in water systems the hydrophilic functional groups exceed the lipophilic groups in the prepolymer that later will form the wall of the capsules when it reacts. And vice versa, in the case of the water in oil emulsions the lipophilic groups exceed the hydrophilic groups in the prepolymer that is used to emulsify and form the wall subsequently.

The main functional groups of the amphiphilic compound are isocyanate (NCO) functional groups, suitable for forming urethane and/or urea type bonds when they react with alcohol (OH) functional groups and amine (NH), respectively. However, another interesting alternative which is not according to the invention, consists in having main functional groups that are silanes instead of isocyanates. These silanes can be obtained, for example, from the reaction of some terminal isocyanates with aminopropyl tri alkylsilane or alkyl aminopropyl trialkyl silane groups (like AMEO, aminopropyl-trimethoxysilane for example, or with the compound Dynasylan ® 1189 sold by Evonik Industries, which is a N-(n-butyl)-3-aminopropyltrimethoxysilane). These functional groups included in structures called hybrid as they are inserted into a base polyurethane-polyurea, form hybrid organic-inorganic prepolymers of polyurea and/or polyurethane (considered organic) with silanes (considered inorganic). These types of hybrid prepolymers react by catalysis (acid, base or organometallic or combined) via the silane functional groups to yield cross-linked glass structures (as in one part of the molecule a glass type structure SiₓO_{y} is formed). In other words, the cross-linking in this case would be between the silane itself and would not require polyamines. These are very stable to hydrolysis and can be interesting in some applications. In fact, the amphiphilic compound could be, in general, mixed, in the sense that its main functional groups can be different from one another. So, for example, in this particular case, the substitution of the NCO groups by silanes could be partial, whereby a hybrid microencapsulate wall would be obtained.

The amphiphilic compound that accumulates in the interphase can be a compound that is added to the first liquid phase, or it can be a compound that is formed in the first liquid phase from a first precursor and a second precursor that react together, i.e. the amphiphilic compound can be added to the already-formed first liquid phase or it can be formed "in situ". In fact, there could be a third precursor (or even more precursors). The first precursor would be the one that shapes the "main chain" of the amphiphilic compound and the second and third precursor are the ones that provide the hydrophilic and hydrophobic side chains, respectively. In fact, the third precursor can be included in the first phase or it can be included in the second phase (both before making the emulsion and after making it). In the event that the third precursor is in the second phase, it is advantageous that, even being soluble in the second phase, it has greater affinity with the first phase. This will help it migrate towards the interphase, where it will be able to react with the product of the reaction between the first precursor and the second precursor, thereby forming the amphiphilic compound directly in the actual interphase.

In the event that the amphiphilic compound is made from precursors, the length of the main chain of the amphiphilic chain "grows" quickly and usually it is no longer possible to have between 4-12 bonds between its main functional groups. However, each of the precursors taking part in the formation of the amphiphilic compound has, in turn, two main functional groups (which are those that will remain included in the main chain of the amphiphilic compound) and a main chain, which is the one extending between the main functional groups of the corresponding precursor and will form, therefore, part of the main chain of the amphiphilic compound. In this respect, it is advantageous that the two main functional groups of the first, second and/or third precursor are separated from each other by between 4 to 12 bonds, preferably between 5 to 10 bonds. This way it is also possible to obtain the advantages of a short main chain indicated above. Preferably each and every one of the precursors making up the amphiphilic compound fulfils this condition.

To form the polymer, the prepolymer can polymerise itself. So, for example, in the event that the main functional groups are isocyanate (NCO), the prepolymer could react with the water in the aqueous phase and it could polymerise with itself. However, in certain cases, it is advantageous that this is not the case, and it can be achieved, following the example that the main functional groups are isocyanate, maintaining the emulsion at a temperature that is low enough to ensure that the isocyanate does not react with the water (or, at least, does not react significantly). So, the second phase comprises a second compound, where the second compound is a polyamine, suitable for reacting with the main functional groups of the amphiphilic compound, to form the polymer that shapes the microencapsulate wall. An advantageous alternative is that the second compound is also amphiphilic, this way it also tends to migrate towards the interphase, and so finds the amphiphilic compound. On the other hand, in the event that the amphiphilic compound is formed "in situ", i.e., in the actual interphase, it is advantageous that this second compound performs the functions of the second precursor indicated above. In this same respect, there could be a third compound which performed the functions of the third precursor and, also, there could be an additional compound that was only responsible for polymerising with the amphiphilic compound formed (i.e. a compound that does not include a hydrophilic or hydrophobic function).

Preferably the first precursor is an isocyanate from the group made up of isophorone diisocyanate (IPDI), hexamethylene 1,6-diisocyanate (HDI) and hydrogenated diphenylmethane 4,4'-diisocyanate (HMDI). In fact, the ideal isocyanate would be a very small diisocyanate such as an ethane or a propane with an NCO function at each end thereof, although they are compounds that are not used on an industrial level. Some of the diisocyanates have the facility of combining to each other forming dimers or trimers, such as for example in the case of HDI. Generally, this invention can use any diisocyanate or triisocyanate out of the ones indicated above as well as any dimer, trimer, tetramer, pentamer, etc. which is derived therefrom. Some examples of these can be dimers of HDI urethdione, trimers of HDI trimethylpropane, trimers of HDI or IPDI isocyanurate, trimmers of HDI or IPDI biuret, HDI allophanates, etc.

Very preferably the isocyanate is an aliphatic isocyanate, which is more stable. It is particularly interesting that the isocyanate be IPDI, or the HDI dimers or trimers, because they are less reactive.

A different alternative, for having amphiphilic compounds with a more acute fattier character, with low HLB, for obtaining water in oil emulsions and capsules in this medium, it would be a fatty isocyanate sold by Cognis as DDI1410 diisocyanate.

Advantageously the second precursor is a hydrophilic compound, and, preferably, the second precursor comprises at least two functional groups suitable for reacting with functional groups of the first precursor. The second precursor preferably has its hydrophilic function in a chain that is sideways with respect to the chain that joins the two functional groups suitable for reacting with functional groups of said first precursor, because, this way, the "main chain" of the amphiphilic compound will be formed from the "main chain" of the first precursor and the "main chain" of the second precursor, and the hydrophilic function will remain in a side position. The second precursor can be a non-ionomeric compound, in which case preferably it is a polyethoxylenate compound with a molecular weight more than 100, very preferable more than 500. In principle, so that a polyethoxylenate compound has water solubility and is equivalent to an ionomeric compound, it must have a relatively high molecular weight (preferably higher than 500). However, the amphiphilic compound can include more than one hydrophilic precursor, in this way their hydrophilic effect is added. Advantageously the polyethoxylenate compound has a molecular weight lower than 5.000, because with greater molecular weights it is probably difficult to adjust with the hydrophobic monomer. If the first precursor is an isocyanate, then this second precursor can be advantageously a mono- or polyalcohol (for example the diol sold under the name of YMER N120 ®) or a mono- or polyamine (for example the monoamine sold under the name JEFFAMINE M1000 ®). So, for example, preferably, the YMER N120 ® would be used when the polyisocyanate is a diisocyanate, to therefore create a linear, difuncional prepolymer with two functional isocyanate end groups (for which the reaction must be made to take place always with excess diisocyanate with respect to the diol); whereas preferably Jeffamine M1000 ® would be used when the polyisocyanate has a functionality greater than 2. Alternatively, the second precursor can be of the ionomeric type, preferably with a carboxylic or sulphonic functional group. In this case, it is advantageous that it has this functional group in a side chain. Advantageously it can be a diol (or diamine) alkyl carboxylic or sulphonic, such as for example dimethylolpropionic acid (DMPA). Other anionomer or cationomer compounds are also possible. The second precursor can also be a mixture of ionomer and non-ionomer compounds.

Preferably the third precursor is a hydrophobic C₈-C₂₂ compound, and comprises at least two functional groups suitable for reacting with functional groups of the first precursor. So, if the first precursor is an isocyanate, the third precursor is preferably an alcohol (or polyol) or an amine (or a polyamine). The third precursor preferably has its hydrophobic function in a chain that is sideways with respect to the chain that links the two functional groups suitable for reacting with functional groups of the first precursor. The hydrophobic function can also be obtained by means of fluoropolymers diols or polysiloxans dioles.

Advantageously the reaction of the first precursor with the second precursor and the third precursor is carried out in the presence of an excess of the first precursor, to prevent the prepolymer from growing excessively and to get the prepolymer to remain reactive with free reactive functional groups.

Preferably the third precursor is a compound from the group made up of fatty diols or diamines C₁₀-C₂₂, and very preferably it is a monoglyceride glycerol, like the monooleate or monostearate glyceryl, a dimerdiol, a cocopropylene diamine or a C₁₆-C₁₈ aminopropyl amine. A tallow oil propylene diamine, or a dimer diamine (Priamine) can also be advantageous. As we will see later, another possibility would be that it is a fatty monoamine or fatty monoalcohol if the initial isocyanate has more than functionality 2. What is left over from functionality 2 is what would be captured with fatty mono amines or mono alcohols and hydrophilic mono amines or mono alcohols, so that in the end we have a reactive prepolymer with an amphiphilic nature and functionality controlled at 2, to prevent prior cross-linking and insolubility while the prepolymer is formed, because there could be sub-reactions when putting polyisocyanates in contact with triamines or with triols before forming the emulsion. In other words, it is not in the method's interest that there is cross-linking before time because the prepolymers would become insoluble (they would precipitate) and would not be able to be emulsified. The tri-functionalisation and cross-linking must emerge after the amphiphilic reactive prepolymer self-emulsifies (in principle linear with functionality 2 or less than 2) and soluble in its phase.

Advantageously following the prepolymer forming reaction an active ingredient is added to said first liquid phase. This "subsquent" addition makes it possible to protect the active ingredient from any "aggression" which it could suffer during the production of the prepolymer. In the case of an organic, disperse phase, the active ingredient will be hydrophobic, and preferably it can be an active ingredient from the group made up of lipophilic vitamins (like retinol, or tocopherol), coenzyme Q10, essential oils, medicinal oils and fragrances. If the disperse phase is aqueous, the active ingredient is preferably a hydrophilic peptide, or a water soluble protein. In fact, generally, it can be any active ingredient in the disperse phase that is soluble (either organic or water, or even it itself can be the disperse phase) and which does not interfere with the reaction forming the interphase of the wall of the microparticles.

Very preferably the active ingredient is an antitumoral drug like paclitaxel, or plitidepsin (sold by Pharmamar). These antitumoral drugs (cytotoxics) are hydrophobics and are not very functionalised and do not interfere with the interphasic reaction.

Advantageously the second compound, i.e. the compound that is added in the second phase, is a diamine, triamine, tetraamine or pentaamine and preferably it is a polyamine from the group made up of ethylene diamine (EDA), diethylenetriamine (DETA), triethylenetetraamine (TETA) and L-lysine. However, it could also be a guanidine, or a sulphonated or carboxylated diamine (or polyamine), which can confer a greater superficial hydrophilic nature apart from cross-linking and closing the active ingredient within the end polymer.

Preferably a fourth precursor is added which, advantageously, comprises a disulphide or an ester in the main chain. Below these cases are analysed in greater detail:
- A first option not according to the invention, is that this fourth precursor comprises an acid group (sulphonate or carboxylate or phosphate) which would be included, for example, in the form of diamine alkylsulphonate (type EPS ® by Raschig or PolyEPS520 ® by Raschig). This fourth precursor would be introduced after introducing the second precursor and the third precursor. Alternatively, instead of introducing this fourth precursor for producing the prepolymer (the amphiphilic compound), this compound can be introduced in the continuous phase, or once the emulsion is made. It could be after or before the prepolymer reacts with the second compound or even together. Generally, it is advantageous to include any compound with a functional group that accelerates the hydrolysis in aqueous means in the presence of esterase type enzymes. This alternative, which introduces ionomeric groups (in principle anionomer, although they could be cationomers also) has a double influence, it can improve the emulsion and stability in water and inhibit the adhesion of the nano or microcapsules to one another, and also it can enhance or accelerate the wall hydrolysis depending on the time. The water attacks the wall more in the presence of ionomers. In fact, a greater hydrophilic part in comparison with the hydrophobic part would also accelerate the hydrolysis of the capsule wall. It would probably also help the walls of the microcapsule to be hydrolysed, for example, by esterases or other hydrolytic enzymes, that are expressed in the proximity of an inflammation (either tumoral or another kind), which would allow selectively releasing the content of the active ingredient in a certain inflamed area of the body.
- A second option is that this fourth precursor comprises a disulphide, which can be made from diamine diakyl disulphides (included at the end) or diol dialky disulphides (included in the prepolymer together with the hydrophilic diol). The disulphide increases the degradation capacity of the capsule wall vis-à-vis the enzymes with thiols in their active centre or glutathione (which is a peptide overexpressed in tumours and inflammations). In both cases, probably there is an overexpression of the same ones around the tumour in the cytoplasm of the affected cells (tumoral cells) due to the exasperated reduction activity by the large amount of organic material in the tumour.
- A third option is that this fourth precursor comprises an ester. In this case, it refers to the presence of ester groups that are particularly sensitive to the hydrolysis in the prepolymer. The presence of esters, which are more hydrolysable functional groups than the urethane or urea, would modulate the hydrolysis of the wall depending on the amount thereof present, again helping the esterases or thiols or other nucleophiles present in the tumoral or inflammed area to hydrolyse the walls of the capsule and release the drug or active ingredient.
- Also, generally, the presence of this type of functional groups would make the unit more biodegradable.

The microencapsulate wall must comply with diverse properties. On the one hand, it must be a "good wall", in the sense that it must be hermetic, but, on the other hand, it must allow the release of the contents when it is convenient. It must also be taken into account that the wall is really a three-dimensional surface and that it is usually made up of a plurality of polymer layers. So, the polymer that shapes the wall must have a certain balance between rigidity and flexibility which, also, must be adjusted for each particular case. However, generally, it can be said that great cross-linking is not advisable. In this respect, the amphiphilic compound (i.e., the prepolymer of the polymer that forms the wall) is mainly difuncional (in the direction of the main functional groups, which are the ones that take part in forming the polymer). There can be a certain degree of trifunctionality (or even greater functionality), but the amphiphilic compound will be "mainly difuncional". The same can be said with respect to the second compound (which is the one that is present in the other phase). An excess of trifunctionality (or, generally, polyfunctionality) when forming the polymer can lead to problems with esters and the formation of gaps or cavities that jeopardise the barrier effect that the microcapsule wall must have. Taking this into account, two preferable strategies can be considered for producing the amphiphilic compound:
- the amphiphilic compound can be obtained from precursors that are all mainly difunctional as well. This is the philosophy of the preferred alternatives indicated in the preceding paragraphs. This way, amphiphilic compounds can be obtained which, for example, can be of the following type:
   diisocyanate-diol-diisocyanate-diamine-diisocyanate the diisocyanate can be a "small" diisocyanate (like IPDI which, on its own, is not amphphilic), the diol can have a hydrophilic side chain (like YMER N120 ®) and the diamine can have a hydrophobic side chain (like Duomeen C ® by Akzo Nobel, which is a fatty C12 diamine). The IPDI with the YMER N120 is too hydrophilic, and so its amphiphilic behaviour improves with the addition of Duomeen C or dimer diol (both have their functional groups (the two amines or the two OH) fairly close, and so the chain is not excessively long). As already mentioned, there can be variants, including some precursor with a greater (for example, by being a mixture of difunctional and trifunctional compounds) or lesser functionality. In short, the interesting point is a reactive amphiphilic prepolymer with linearity and some (albeit little) trifunctionality, so that it is self-emulsifying. Generally, it is advantageous that the space between the hydrophilic and hydrophobic part is not large. It is also interesting that the polymer that shapes the wall has groups resistant to the water, oil or organic solvent used, as is the case of the ureas and urethanes.
- another different strategy for obtaining the amphiphilic compound is from a first precursor (which, preferably is an isocyanate) with a high functionality (greater than 3) and forming the amphiphilic compound by joining the remaining precursors directly to the first precursor, and not forming a chain as in the case above. In this case, the remaining precursors have low functionality (smaller than 2) to prevent excessive cross-linking in the unit. Generally, a certain molecule has to have a degree of functionality that must necessarily be a whole number. However many compounds are really a mixture of similar, but not identical, molecules. These compounds can have functionality values that are not whole numbers, because they are, in fact, the average value of the functionalities of each component. In this respect the functionalities indicated in this specification and claims must be understood. A preferred way of implementing this alternative is by using a polyisocyanate (like a tetramer, or hexamethylene diisocyanate trimer allophanate, such as for example the compound Bayhydur VPLS2319 ® (which is a polyisocyanate based on hexamethylene diisocyanate, and is sold by Bayer), which has a functionality of 3.8 and is already hydrophilised) to which an amine or monofunctional fatty alcohol is added (preferably linear or branched C10-C22, and very preferably linear C12-C18) (to provide the hydrophobic function) and functional amine or alcohol (such as for example Jeffamine M1000) (to provide the hydrophilic function), although other precursors are also possible, like glycolic, glycine, taurine, monomethyl ethers of polyethoxylated alcohols, etc. The resulting amphiphilic compound will still have (or between 2 or 3) free isocyanates with which it will be able to form the polymer that shapes the wall of the microcapsules.

A second prepolymer is added to the first phase, where the second prepolymer has at least two main functional groups that react in the subsequent polymerisation for producing the polymer, where the second prepolymer is more hydrophobic than the amphiphilic compound if said first phase is organic, or the second prepolymer is more hydrophilic than the amphiphilic compound if said first phase is aqueous. In fact, this way, this second prepolymer will tend to arrange itself as a second internal layer in the wall of the microcapsules, given its greater affinity to the first phase. This second prepolymer will react also with the second compound thereby forming a wall with two layers, which confers greater robustness in the case of any possible subsequent liophilising, redispersion processes, etc., and, also, greater encapsulating power is achieved for the active ingredients included inside it. The two layer structure also means better control over the amount of functionalisations and the degradability of the particle. This second prepolymer can be, in turn, a mixture of two or more components, for example, in the case of an O/W emulsion they are preferably a diisocyanate (like isophorone diisocyanate) and a polyisocyanate (like Bayhydur 3100 ®, which is a hydrophilic, aliphatic polyisocyanate based on hexamethylene diisocyanate sold by Bayer).

Advantageously, the polymer comprises a functionalising group, such as for example a tumour cell directionaliser. The functionalising group has preferably two main functional groups in this way the functionalising group remains integrated in the main chain of the polymer or amphiphilic compound. The functionalising group can be preferably a monoclonal antibody or include a monoclonal antibody. Advantageously the polymer comprises a peptide, which can be incorporated directly into the amphiphilic compound. The peptide is preferably in the main chain and, if the wall has two layers, it is preferably in the outer layer or between the two layers, because it has to be able to interact with the outside medium. In fact, a particularly interesting solution in the invention is when this peptide is able to interact with overexpressed proteins outside the tumour cells (the integrins alpha beta). In this respect, it is particularly advantageous that the peptide be c-(RGDfK). Another preferred interaction strategy between particles and cancerous cells is carried out by means of folic acid (FA) incorporated in the same way as c-(RGDfK). The FA interacts with folate receptor proteins that are overexpressed in numerous cancer cells.

Generally, it may occur that the functionalising element (the peptide, the monoclonal antibody, or the functionalising molecule in general) does not have two main functional groups. In this case, the functionalising element can be made to react with a linker (linking element), such as for example a di- or polyisocyanate. This way, the link between the functionalising element and the linker makes it possible to obtain the functionalising group indicated above. So, preferably, the peptide is made to react with a di- or polyisocyanate (such as for example Bayhydur 3100 ®) and the reaction product is added to the first phase, which is the organic phase. Usually the amount of water present in the aqueous solution of the peptide is not enough to cause the complete emulsion, including the phase inversion. In these cases, subsequently more water is added until the phase inversion takes place and then an aqueous solution continues to be added to the second compound (which preferably is a diamine and very preferably is DETA).

Generally, it is advantageous to add the second compound once the emulsion has already formed, including the phase inversion. In fact, when starting to add the second compound the formation of the microcapsule wall starts to form. Therefore, it is of interest that the microcapsules are already fully formed when the second compound is added. This is particularly important when the reactivity of the second compound is high, such as for example in the case of DETA with isocyanates.

An advantageous embodiment of the method according to the invention when the emulsion is an O/W emulsion, comprises a stage of adding a polar, volatile organic solvent to the first phase, before stage [a], and a solvent evaporation phase, after stage [b]. This makes it possible to obtain very small size microencapsulates and in relatively high concentrations. Preferably the solvent is acetone, methyl ethyl ketone or tetrahydrofuran, and very preferably it is acetone or tetrahydrofuran. If it is acetone, advantageously the amount of acetone added is smaller than 20% by weight of the amount of water in the second phase, whereas in the case of the methyl ethyl ketone advantageously the amount of methyl ethyl ketone added is less than 15% by weight of the amount of water in the second phase.

Preferably the polymer or the amphiphilic compound is amphoteric. Advantageously at least one of the precursors used to form the amphiphilic compound is ionic. So, preferably the amphiphilic compound includes protonated tertiary amines or quaternary ammoniums (or, generally, a cationomer) combined with anionomers in the same main chain. Therefore it is advantageous that some of the precursors used to form the polymer have two main functional groups and, also, a cationomer and/or an anionomer so that, once the polymer or amphiphilic compound is formed, this is amphoteric.

The aim of the invention is also a microencapsulate obtained according to the method of the invention.

Preferably the microencapsulate comprises, inside, an antitumoral active ingredient which, advantageously, is paclitaxel or plitidepsin.

Another advantageous solution is obtained when the microencapsulate according to the invention comprises, inside, an antibacterial agent. The bacterial agent is preferably a hydrophobic antibacterial, and very preferably it is a hydrophobic antibacterial agent from the family of macrolides. Particularly, these agents can be roxithromycin or clarithromycin.

The aim of the invention is also a cosmetic or pharmaceutical composition characterised in that it comprises a microencapsulate according to the invention.

Finally, the aim of the invention is also a new use of a microencapsulate according to the invention, specifically for superficially coating a biocompatible material, preferably a biocompatible metal and very preferably titanium or its alloys.

### Brief description of the drawings

Other advantages and characteristics of the invention are appreciated from the following description, wherein, some preferable embodiments of the invention are described in a non-limiting manner, with reference to the accompanying drawings, in which:
Figs. 1 to 4, size distribution of the capsules in Examples 1, 2, 3 and 4, respectively.
Figs. 5 to 9, SEM images of the capsules in Example 4.
Figs. 10 and 11, SEM and TEM images, respectively, of the capsules in Example 13.
Figs. 12 and 13, SEM and TEM images, respectively, of the capsules in Example 14.
Figs. 14 and 15, TEM images of a degrading of the capsules in Example 19, filtered 0.22 µm.
Figs. 16 and 17, TEM images of a degrading of the capsules in Example 19, filtered 0.45 µm.
Figs. 18 and 19, TEM images of another degrading of the capsules in Example 19, filtered 0.22 µm.
Figs. 20 and 21, TEM images of a degrading of the capsules in Example 18, filtered 0.22 µm.
Fig. 22, TEM image of the capsules in Example 28.
Fig. 23, TEM image of the capsules in Example 31.
Fig. 24, TEM image of the capsules in Example 35.

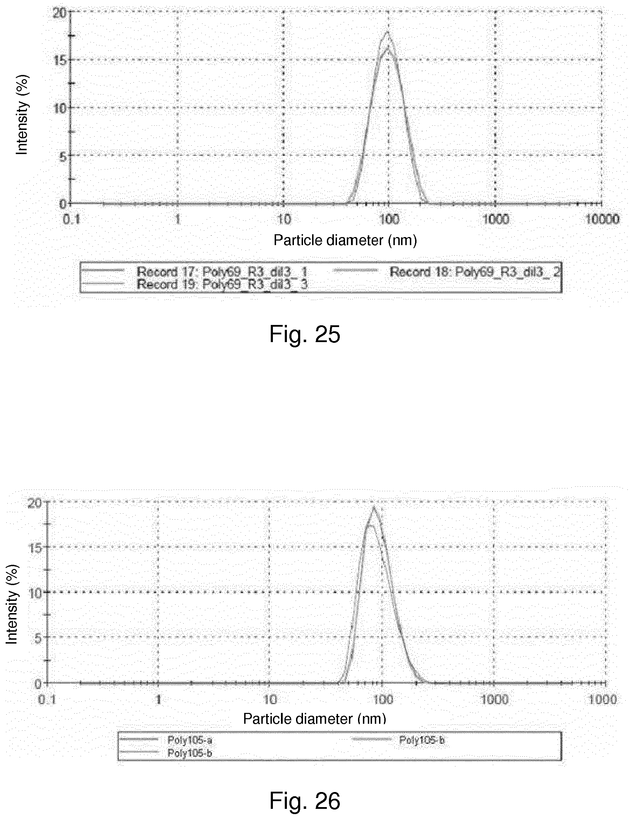

Figs. 25 and 26, size distribution of the capsules in Examples 18 and 34, respectively.
Figs. 27a to 27d, cytotoxicity study of nanocapsules relative to Example 28.
Fig. 28, biodistribution study in athymic mice without a tumour in the nanocapsules in Example 28 loaded with DiR.
Fig. 29, reaction diagram and theoretical representation of the ideal structure of the nanocapsule in Example 28 loaded and functionalised following Examples 31-33.
Figs. 30 and 31, size distribution of the capsules in Example 36.
Figs. 32 and 33, TEM images of the capsules in Example 36.
Figs. 34 and 35, evolution of the fragrance intensity over time of the capsules in Example 36.
Fig. 36, TEM image of the capsules in Example 37.
Fig. 37, diagram of the linker between the cRGDfK peptide and a microcapsule in Example 38.
Fig. 38, diagram of the amphiphilic prepolymer (AMPHYL) in Example 38.
Fig. 39, diagram of the hydrophobic prepolymer (HYFOB) in Example 38.
Fig. 40, theoretical representation of the ideal structure of the nanocapsule in Example 38.
Figs. 41 to 43, evolution over time of the degrading of the microcapsules in Example 38 due to the action of glutathione.
Fig. 44, diagrammatic representation of the functionalising process in Example 39.
Fig. 45, SEM image of the functionalised titanium surface in Example 39.
Fig. 46, fluorescence spectrum of the mixture of DiL and DiO in Example 41 after 84 hours at 37 ºC and the corresponding fluorescence spectrum of the mixture of nanocapsules 2.5 hours after adding GSH.
Fig. 47, representation of the FRET ratio in Example 41, calculated as Iₐ/(I_{d} + Iₐ), where Iₐ e I_{d} are the fluorescence intensity of the acceptor (Dil) and donor (DiO), respectively against time.

### Examples

### Products used.

- Isophorone diisocyanate (IPDI),
- Ymer N120 ® (difunctional linear polyethylene glycol monomethyl ether) by Perstorp,
- Pripol 2033 ® (Dimerdiol) by Uniqema,
- Crodamol GTCC® by CRODA (which is a caprylic/capric triglyceride),
- olive oil,
- Lavandín ® and Aromatic Meadow ® fragrances,
- Tea tree oil,
- Retinol 10S ® by Bayer (retinol at 10% in soya oil),
- Paclitaxel by Fujian South Pharmaceutical,
- Plitidepsin by Pharmamar,
- c-(RGDfK) by PCB,
- Folic Acid (FA) by Aldrich,
- Roxithromycin by Aldrich,
- Clarithromycin by Aldrich,
- Clear Blue DFSB-C0 by Risk Reactor,
- Ethylenediamine propyl sulphonate (EPS) by Raschig,
- NaOH by Panreac,
- BYK-028 ® by BYK,
- Ethylenediamine (EDA),
- Diethylenetriamine (DETA),
- Triethylenetetramine (TETA) by Huntsman,
- Hexamethylene diamine (HMDA) by Panreac,
- Duomeen C ® (dodecylamine propyl amine (coconut)) by Akzo Nobel,
- Duomeen T ® (C16-C18 amino propylamine) by Akzo Nobel,
- TAP 100D (N-octadecyl- 1,3 -propanediamine, by Clariant, it is equivalent to Duomeen T by Akzo Nobel),
- Desmodur N3600 -1,6-hexamethylene diisocyanate, polymeric form
- Jeffamine M1000,
- Triameen C (N-cocoalkyl dipropylene triamine),
- LAP 100D (dodecyl amino propylamine (coconut), by Clariant, equivalent to Duomeen C by Akzo Nobel),
- Triameen T (N-tallowalkyl dipropylene triamine),
- Priamine 1074 (dimer diamine from C36 dimer acid) by Croda,
- DDI1410 disocyanate by Cognis (fatty isocyanate derived from dimer acid),
- Bayhydur 3100 ® (aliphatic hydrophilic polyisocyanate based on hexamethylene diisocyanate) by Bayer,
- Triethylamine by Aldrich,
- 2,2-dithiodiethanol (DEDS) by Aldrich,
- cystamine dihydrochloride by Aldrich,
- L-lysine by Aldrich,
- dibutyltin dilaurate (DBTL),
- tetrahydrofuran (THF),
- CTP (cell targeting peptide), such as for example c-(RGDfK))

### Example 1. Encapsulation of olive oil.

In a 700 ml reactor 10.53 g of IPDI (which performs the function of the first precursor), 6.38 g of Dimerdiol (which performs the function of the second precursor), 20.38 g of olive oil and 20.83 g of Ymer (which performs the function of the third precursor) were loaded. The reaction was carried out at 160 rpm, at 85ºC and with nitrogen.

After 2 hours reaction time, the reactor was cooled to below 25ºC and 3 drops of BYK-028 antifoaming agent were added, the emulsion was made at 400 rpm with 250.00 g of water, adding it slowly on top of the amphiphilic prepolymer.

Once the emulsion was complete, 3.28 g drops of EPS (which performs the function of the fourth precursor), 0.36 g of NaOH and 10.20 g of water were added. After 20 minutes 0.43 g of DETA (which performs the function of the second compound) were added. The EPS was added to combine with the prepolymer and make it more hydrophilic, but essentially it was done to include a sulphonate that was a weak point in the chain. It also allowed reducing the particle size a little. As already mentioned above, generally disulphide or labile ester groups can be added expressly, so that they are a weak point for breaking (for example, at the estearases). In the proximity of the tumours there is much reducing material that breaks the disulphide bridge. (See also Example 19).

A good white bluish emulsion was obtained. Using an optical microscope capsules sized smaller than 4 µm were observed. Using Light Scattering (DLS, Dynamic Light Scattering), it was confirmed that the capsules were sized between 0.4 and 4 µm.

### Example 2. Encapsulation of an Aromatic Meadow C55593P fragrance by Lucta (reference example).

In a 700 ml reactor 10.53 g of IPDI (first precursor), 6.40 g of Dimerdiol (second precursor), 20.36 g of Aromatic Meadow and 15.52 g of Ymer (third precursor) were loaded. The reaction was carried out at 160 rpm, at 85ºC and with nitrogen. After 2 hours reaction time, the reactor was cooled to below 25ºC and 3 drops of BYK-028 antifoaming agent were added, the emulsion was made at 400 rpm with 250.00 g of water.

Once the emulsion was complete, 3.17 g of EPS (fourth precursor), 0.35 g of NaOH and 10.00 g of water were added. After 20 minutes 0.79 g of DETA (second compound) were added.

A good white bluish emulsion was obtained. Using an optical microscope capsules sized smaller than 4 µm were observed. Using Light Scattering, it was observed that the capsules were sized between 0.2 and 3 µm. The population was distributed between 0.2 and 0.4 µm and again between 1 and 3 µm.

### Example 3. Encapsulation of Retinol 10S.

Example 1 was repeated replacing the 20.38 g of olive oil with 20.34 g of Retinol 10S.

In this case a good emulsion was also obtained, but with capsules sized smaller than 10 µm. Using Light Scattering, it was observed that the capsules were sized between 0.15 and 10 µm. Most of the population was between 1 and 3 µm.

### Example 4. Encapsulation of the Aromatic Meadow C55593P fragrance by Lucta (reference example).

Example 2 was repeated with TETA instead of DETA. By using YMER and DIMERDIOL, together they form a very soft capsule wall. In this case, using TETA allowed for more intensive cross-linking. In those cases where with TETA the cross-linking was excessive, DETA could be used.

In a 700 ml reactor 10.52 g of IPDI, 6.57 g of Dimerdiol, 20.29 g of Aromatic Meadow and 15.37 g of Ymer were loaded. The reaction was carried out at 160 rpm, at 85ºC and with nitrogen. After 2 hours reaction time, the reactor was cooled to below 25ºC and 3 drops of BYK-028 antifoaming agent were added, the emulsion was made at 400 rpm with 200.00 g of water. Once the emulsion was complete, 3.50 g of EPS, 0.39 g of NaOH and 10.50 g of water were added. After 20 minutes 1.00 g of TETA was added.

A good emulsion was obtained with capsules sized less than 4 µm. Using Light Scattering, it was observed that the capsules were sized between 0.3 and 4 µm. Most of the population was between 0.3 and 1 µm.

### Example 5. Encapsulation of the Lavandín 80101 fragrance by Chemir (reference example).

Example 4 was repeated replacing the 20.29 g of Aromatic Meadow with 20.66 g of Lavandín fragrance.

In this case too a good emulsion was obtained, with capsules sized smaller than 4µm.

### Example 6. Encapsulation of Tea Tree Oil (reference example).

Example 4 was repeated replacing the 20.29 g of Aromatic Meadow with 20.45 g of Tea Tree Oil.

In this case too a good emulsion was obtained with capsules sized smaller than 4 µm.

### Example 7. Encapsulation of Tea Tree Oil (reference example).

Example 6 was repeated but with half the polymer.

In a 700 ml reactor 5.28 g of IPDI, 3.26 g of Dimerdiol, 20.45 g of Tea Tree Oil and 7.77 g of Ymer were loaded. The reaction was carried out at 160 rpm, at 85ºC and with nitrogen. After 2 hours reaction time, the reactor was cooled to under 25ºC and 3 drops of BYK-028 antifoaming agent were added, the emulsion was made at 400 rpm with 155.00 g of water. Once the emulsion was complete, 1.68 g of EPS, 0.19 g of NaOH and 5.00 g of water were added. After 20 minutes 0.49 g of TETA were added.

A good emulsion was obtained and the distribution of the capsule size was between 4 and 0.1 µm.

### Example 8. Encapsulation of Tea Tree Oil (reference example).

Example 7 was repeated but increasing the cross-linker (from 0.49 g of TETA to 0.72 g of TETA).

In this case too a good emulsion was obtained but with capsules that were not so spherical, and the capsule diameter between 5 and 0.1µm.

### Example 9. Encapsulation of Paxlitaxel (reference example).

In a 700 ml reactor 10.53 g of IPDI, 6.58 g of Dimerdiol, 20.00 g of Crodamol GTCC and 15.39 g of Ymer were loaded. The reactor was carried out at 160 rpm, at 85ºC and with nitrogen. After 2 hours reaction time, the reactor was cooled to under 25ºC and 0.64 g of Paclitaxel, 3 drops of BYK-028 antifoaming agent were added and the emulsion was made at 400 rpm with 200.00 g of water. Once the emulsion was complete, 3.10 g of EPS, 0.68 g of NaOH and 10.00 g of water were added. After 20 minutes 1.00 g of TETA was added.

A good emulsion was obtained with capsules between 2 and 0.1 µm.

### Example 10. Encapsulation of Crodamol GTCC (reference example).

In a 700 ml reactor 10.54 g of IPDI, 6.57 g of Dimerdiol, 20.29 g of Crodamol GTCC and 19.95 g of Ymer were loaded. The reaction was carried out at 85ºC and with nitrogen. After 2 hours reaction time, the reactor was cooled to under 25ºC. The emulsion was made at 15,000 rpm by adding the prepolymer on to 200,00 g of water, once the emulsion was complete, 2.64 g of EPS, 0.29 g of NaOH and 10.50 g of water were added at 5 000 rpm. After about 3 minutes, 0.75 g of TETA were added.

A good emulsion was obtained, in the optical microscope capsules were observed between 0.1 and 4 µm. It was observed also that there was a lesser amount of large capsules than in other Examples.

### Example 11. Encapsulation of Crodamol GTCC (reference example).

This product is the same at Example 10 but with EDA instead of EPS. The EDA is less hydrophilic than the EPS.

A total of 10.78 g of IPDI, 6.67 g of Dimerdiol, 21.13 g of Crodamol GTCC and 20.36 g of Ymer were loaded. The reaction was carried out at 85ºC and with nitrogen. After 2 hours reaction time, the reactor was cooled to 25ºC, 2 drops of BYK-028 antifoaming agent were added and the emulsion was made with 200.00 g of water in the Ultra-Turrax at 15.000 rpm. Once the emulsion was complete, 0.39 g of EDA were added to 10.00 g of water with mechanical stirring. After 30 minutes 0.52 g of TETA were added.

A good white bluish emulsion was obtained, in the optical microscope capsules were observed sized 0.9 µm but also some agglomerated capsules. Using Light Scattering, it was observed that the capsules were sized smaller than 0.9 µm. Most of the population was 246 nm.

### Example 12. Encapsulation of Crodamol GTCC (reference example).

This product is the same as Example 11 but with less polymer.

A total of 5.85 g of IPDI, 3.35 g of Dimerdiol, 21.20 g of Crodamol GTCC and 10.17 g of Ymer were loaded. The reaction was carried out at 85ºC and with nitrogen. After 2 hours reaction time, the reactor was cooled to 25ºC, 2 drops of BYK-028 antifoaming agent were added and the emulsion was made with 200.00 g of water in the Ultra-Turrax at 15,000 rpm. Once the emulsion was complete, 0.39 g of EDA were added to 10.00 g of water with mechanical stirring. After 30 minutes 0.52 g of TETA were added.

A good white bluish emulsion was obtained, in the optical microscope capsules were observed that sized 0.9 µm but there were also some agglomerated capsules.

### Example 13. Encapsulation of Crodamol GTCC (reference example).

This Example used the system of creating the amphiphilic polymer at the same time as making the emulsion.

The following were mixed in a beaker:
5.26 g of IPDI (first precursor)
11.45 g of Ymer (second precursor)
6.05 g Crodamol GTCC

The following were mixed in another beaker:
3.15 g of Duomeen C (third precursor)
210.00 g of water

The organic solution was added to the aqueous one and it was stirred at 15,000 rpm for 30 minutes.

A good white bluish emulsion was obtained, in the optical microscope capsules were observed smaller than 450 nm. The product was filtered with a syringe and 0.45 µm filter, and 88.2% of the capsules were smaller than 450 nm.

Duomeen, in spite of being hydrophobic, has sufficient dispersibility in water to put it in the continuous aqueous phase. However, as it is more hydrophobic than hydrophilic, it ends up going to the disperse, oily phase and there it reacts and is incorporated into the prepolymer. This allows carrying out the reaction to introduce the hydrophobic part after making the emulsion from the the hydrophilic prepolymer IPDI-YMER part, in order to form the amphiphilic prepolymer just in the interphase. Then the capsule is closed fully (crosslinked) with the polyamines that wear the NCO that remain from the amphiphilic prepolymer formed in the actual interphase. Generally, this Duomeen will have to be introduced into the prepolymer as in the case of the dimer diol, but a solvent is required because if not it will not work well because of its insolubility in the oil medium that is used. Duomeen, as it is a diamine, forms a diurea. The dimer diol is a different case, forming two urethanes. In certain cases it is solid enough, has a sufficiently high Tg, creates a sufficient barrier effect and is stable enough to withstand the capsule contents, which is harder to achieve with a prepolymer based on dimer diol polyurethanes- YMER IPDI. In this case DETA is not added, or any diamine or small, hydrophilic polyamine, and instead it is left to react with the water.

Alternatively the Duomeen could have been introduced in the prepolymer with a suitable solvent as in the case of the dimer diol. And also it is possible to use a small amount of DETA to close (crosslink) and ensure the system's strength. However, the results seem to be more repeatable than with the interphasic system in this Example. In the case of the dimer diol-IPDI-YMER, the formed wall of the capsule is more sensitive because the prepolymer has greater hardness (lower Tg) and is more soluble in the phases, therefore it is good to add DETA or TETA type polyamines to confer hardness and a barrier effect, etc.

### Example 14. Encapsulation of Crodamol GTCC (reference example).

This Example used the system of creating the amphiphilic polymer at the same time as making the emulsion.

A total of 1.71 g of IPDI, 5.04 g of Ymer N120 and 2.64 g of Crodamol GTCC were loaded. The reaction was carried out at 85ºC and with nitrogen. When NCO=1.372% was reached, the reactor was cooled and 211.60 g of acetone were added. In another reactor 0.31 g of Duomeen C were added in 422.98 g of water. The acetonic phase was added on top of the aqueous phase at 400 rpm. Finally, 0. 08 g of DETA were added to 5.00 g of water and the acetone evaporated.

A very good emulsion was obtained, in the optical microscope capsules were observed with a diameter smaller than 0.5 µm. % solids=2.00%

The product was filtered with a syringe and 0.45 µm filter, and 81.5% of the capsules were smaller than 450 nm.

### Examples 1 to 14 - Analysis and results

In all the Examples a Titromatic 2S tritrator by Crison was used to check the NCO percentage of the prepolymer. Also the end of the reaction was checked using IR, observing the characteristic signals of the polyureas (1650-1500 cm⁻¹) and the absence of signal due to the NCO (2270cm⁻¹).

The morphology and size of the capsules obtained in the Examples were studied using an optical microscope and SEM.
The size distribution of the capsules was studied using Light Scattering.

In all the Examples emulsions stable over time were obtained with capsules sized smaller than 5 µm, except in Example 3 where capsules smaller than 10 µm were obtained, and in Examples 13 and 14 where capsules smaller than 0.45 µm were obtained.

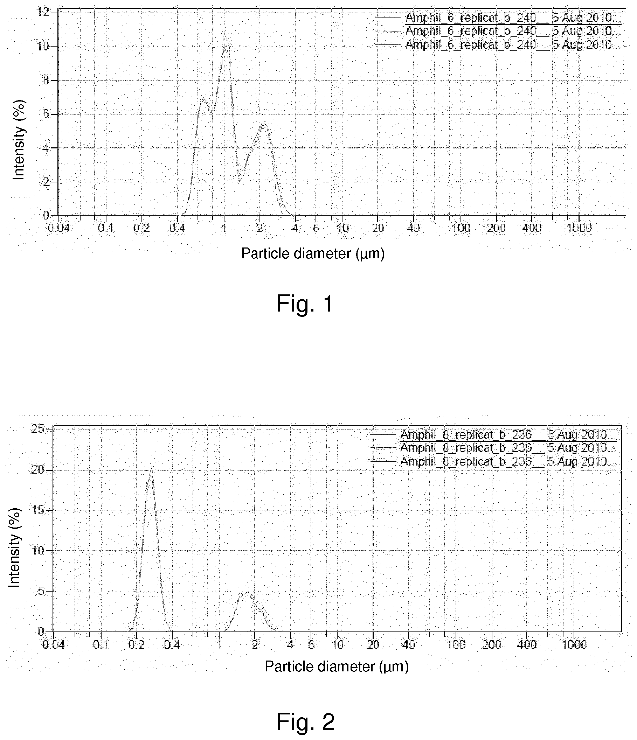

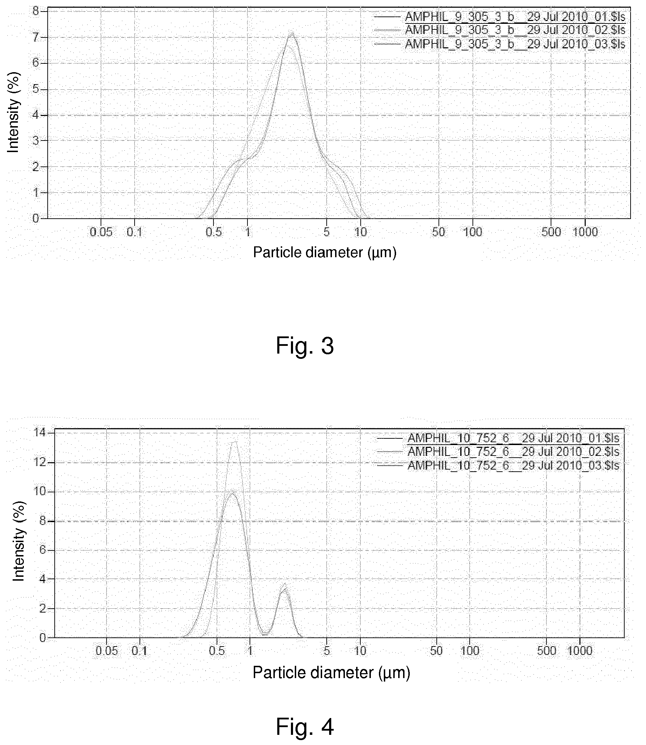

The graphs in Figs. 1 to 4 show the size distribution of the capsules in Examples 1, 2, 3 and 4, respectively.

In these graphs it can be observed that most of the capsules are under the micron (except Example 3) because the % volume is much greater in this area. Using filtration, the larger capsules can be separated and even more stable emulsions can be obtained and with a more uniform distribution.

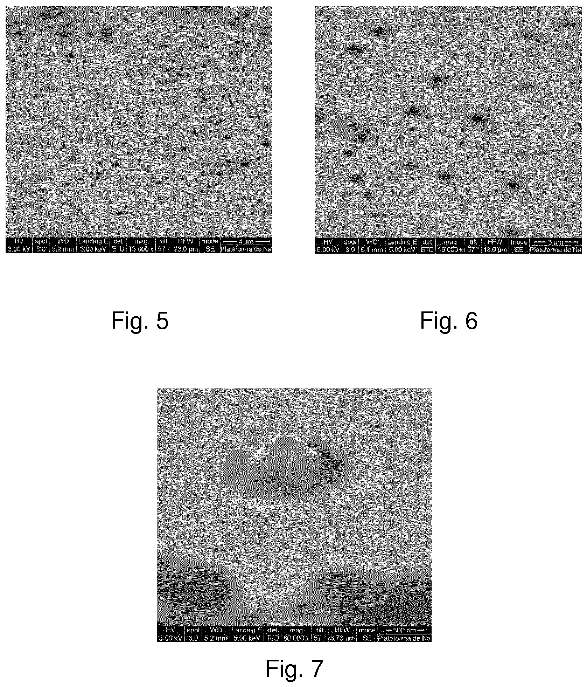

SEM was used to study the morphology and size of the capsules obtained in Example 4 (see Figs. 5 to 7). The images of the capsules were obtained after diluting them with Millipore 1/100 water and coating them with gold.

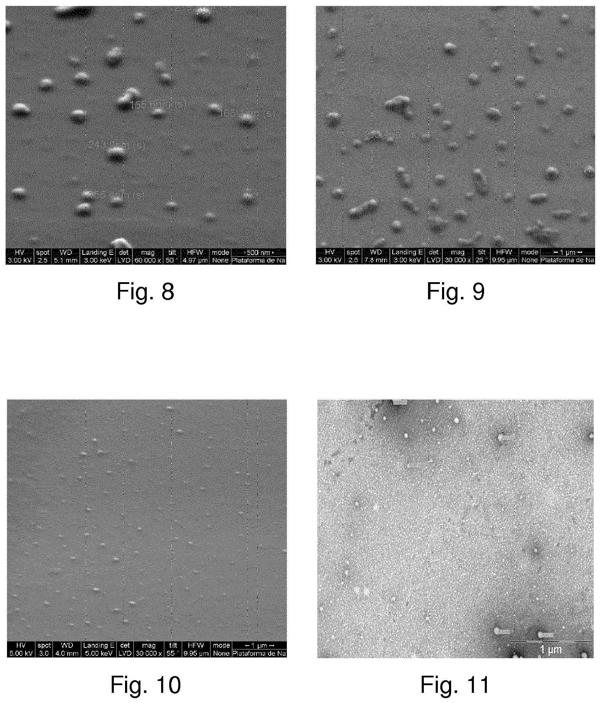

Using 0.45 µm filters the diluted capsules were filtered to obtain clearer images and to remove the capsules sized larger than 0.45 µm (see Figs. 8 and 9).

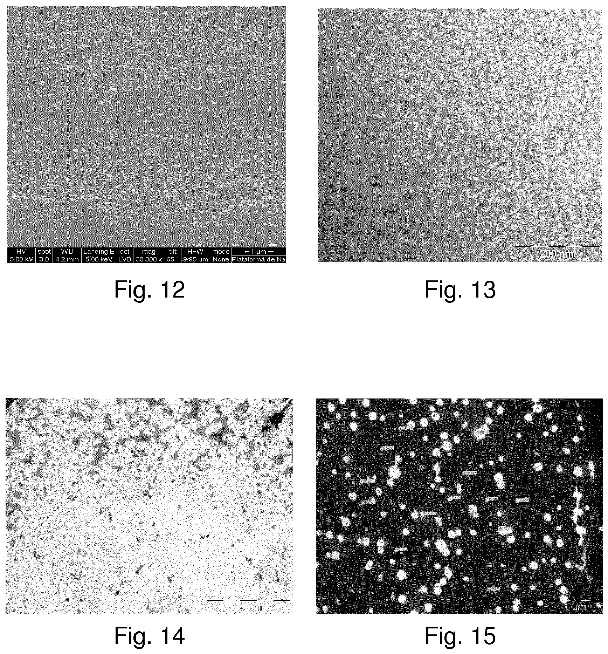

Figs. 10 and 11 show the capsule images obtained using SEM (Scanning Electron Microscope) and TEM (Transmission Electron Microscope) respectively in Example 13, and Figs. 12 and 13 show the capsule images obtained using SEM and TEM respectively in Example 14.

### Examples 1 to 14 - Conclusions

With this amphiphilic system and with different types of processes, various compounds can be encapsulated obtaining capsules sized less than 5 µm if using the system wherein the amphiphilic polymer is formed before forming the O/W emulsion. Generally, preferably the emulsion is created by phase inversion by adding water to the prepolymer (generally, the continuous phase to the disperse phase). With stability and without using external emulsionants, and less than 450 nm if using the system wherein the amphiphilic polymer is formed during the emulsion.

In both cases the emulsions obtained are very stable over time, the capsules do not join together and they are stable with respect to biological fluids, etc.

The stability of the products obtained in Examples 11, 13 and 14 was checked in different media: saline, PBS (Phosphate Buffered Saline) and blood plasma. The results were as follows:

| Nanocapsules | Stability after 1 week | | | Stability after 1 month | | |
|---|---|---|---|---|---|---|
| | SS | PBS | Blood plasma | SS | PBS | Blood plasma |
| Example 11 | stable | stable | stable | stable | stable | unstable |
| Example13 | stable | stable | stable | stable | stable | stable |
| Example 14 | stable | stable | stable | stable | stable | unstable |

It must be taken into account that the products known in the state of the art, with normal emulsifying agents outside the wall, are not stable in these media and precipitate.

### Example 15 (reference example).

A total of 19.02 g of Crodamol GTCC + 7.56 g Desmodur N3600 + 10 g methyl ethyl ketone (MEK) were added to a beaker. Slowly, with 50% magnetic stirring, first 8.34 g Jeffamine M1000 + 5 g MEK were added, then 2.13 g DuomeenT + 5 g MEK with 75% stirring. On this basis four tests were conducted with this prepolymer:
15-1 → 15 g of the product were added on to 90 g of water with 75% stirring, dropwise. The water contained 0.16 g of EDA.
15-2 → Dropwise, 88 g of water were added on to 15 g of the product. When the phase inversion was carried out, 2 g of water + 0.16 g of EDA were added.
15-3 → 15-1 was repeated, but by injection, not by adding water dropwise.
15-4 → Imitation of the ketonic method, 0.6 g of prepolymer were put in 20 ml acetone, and they were injected into 80 ml of water + 0.22 g EDA.

### Results:

15-1 → The capsules were dented. There seemed to be capsules inside capsules. Size 2-16 microns.
15-2 → The capsules had a much more regular shape. The emulsion was stable. Capsule size approx. 2-6 microns.
15-3 → It worked out the same as 15-1, and it seemed that the injection was not a vital aspect, providing that the addition was carried out slowly.
15-4 → A transparent emulsion was obtained, with bluish tones. No capsules were observed with the optical microscope. SEM images showed capsules with a wide size distribution, from 7 to 100 nm, with an average of 20 nm. (*Amphil HLB12)*

### Example 16 (reference example).

An experiment similar to the one above, but using polymeric MDI (polymeric diphenylmethane diisocyanate (or methylene diphenyl diisocyanate)).

On to 21 g Crodamol GTCC + 4.90 g polymeric MDI + 5 g MEK first of all a mixture of 9.73 g Jeffamine M1000 + 10 g MEK was added, and later, 2.13 g Duomeen T + 5 g MEK, all dropwise, with 50% mechanical stirring. Subsequently, 0.6 g of this product were added by injection into 20 ml acetone onto 40 g water + 1 drop of DETA, the injection lasted 1'. The emulsion was white-bluish and very stable (it did not separate or foam). The capsules could not be seen with the optical microscope.

### Example 17 (reference example).

Encapsulation using aromatic /aliphatic diisocyanate with triamine.

A total of 16.67 g Crodamol GTCC + 4.89 g TMXDI ((tetra methyl xylylene diisocyanate) + 5 g MEK were weighed.

Dropwise, with 50% magnetic stirring, 2.19 g TriameenC + 5 g MEK were added. Subsequently, 6.95 g Jeffamine M1000 + 8 g MEK were added.

A total of 3 tests were conducted with this prepolymer using the methodology in Example 14, by injection:
A → 2.2% of solids, 4 drops of DETA in H2O.
B → 2.2% of solids, 1 drop of DETA in H2O.
C → 10% solids, 0.5 g of DETA in H2O.

### Results:

A → Capsules measuring 2 microns, very narrow size distribution. Regular capsule shape.
B → The same as A, but the shape of the capsules was much more irregular.
C → They came out joined together, but the distribution and size was the same as A.

### Example 18. Encapsulation of Crodamol GTCC (reference example).

Similar to Example 14 but the method was carried out in two stages:
1) A total of 6.01 g of Crodamol GTCC, 11.79 g of Ymer N120 and 5.72 g of IPDI were loaded. The bath was set at 85ºC with N₂ current. It was left to react during two and a half hours. When the theoretical NCO level was reached, the reactor was cooled to 50ºC and its contents was dissolved with 12.67 g dry MEK.
2) A total of 1.66 g of Duomeen C were dissolved in 25.50 g of MEK and added to the reactor. When the second theoretical NCO was reached, the system was cooled with a bath of water and ice and the emulsion was made by adding cold water dropwise. Then 0.42 g DETA dissolved in 3.76 g of water was added. Finally the MEK evaporated.

### Product appearance:

White liquid and very small capsules mainly with a size smaller than a micron. The product was filtered very easily using micro filters with pores sized 0.450 and 0.200 µm.

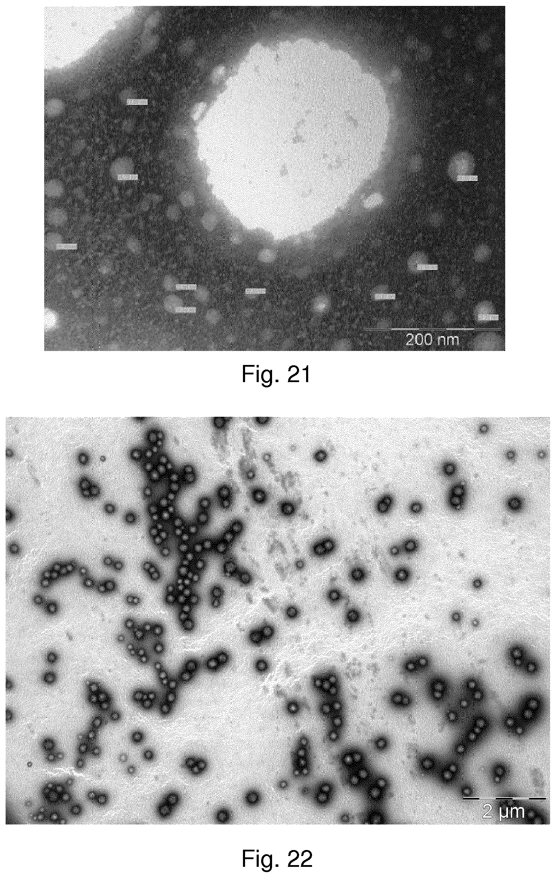

The TEM and SEM product was observed, and nanocapsules were observed up to 20 nm in size. Also various repetitions of this product were made and the results were approximately repeatable insofar as the particle size. A DLS analysis was conducted and a main average population of 100 nm in size was observed. Fig. 21 shows an image obtained with the SEM where agglomerates of nanocapsules could be observed sized smaller than 100 nm.

### Example 19. Encapsulation of Crodamol GTCC

This Example incorporates a disulphide bridge in the capsular wall. The fact that there is a disulphide in the chain favours the wall degradation with the glutathione and other reducing agents inside and around the tumour cells.

A total of 5.562 g IPDI + 10.338 g Crodamol GTCC + 12.5256 g dry Ymer were added to the reactor at an internal reactor temperature of 63 ºC. When the theoretical NCO % was reached a 520 µl (0.655g) micropipette was used to add 2.2-dithiodiethanol. It was observed that the 2.2-dithiodiethanol was denser and the stirring had to be optimum so that different phases did not form during the reaction. When the theoretical NCO% was reached the temperature was lowered to 50 ºC and 1.4368 g LAP 100D solublised in 20 g of dry MEK were added. After 20 minutes the reactor was cooled to 10 ºC. A clear thickening of the mixture was observed. A total of 3 drops of BYK-028 antifoaming agent were added. Next the dropwise addition of 230 ml of water cooled to 15 ºC started. When the phase inversion took place 155 µl (0.1479 g) of DETA was added in the addition funnel continuing with the more frequent dropwise addition of the rest of the milliQ water + DETA.

An IR was done before the emulsification to check the NCO presence in reaction. It was left under stirring and cooling to 10 ºC for 2 more hours after the emulsification.

White-bluish, even appearance. Stable over time, it was stored in the fridge. It was filtered easily in the 450 nm filter and also in the 220 nm one.

### Degradation tests of the polymer functionalised with a disulphide bridge against the reducing agent glutathione (GSH)

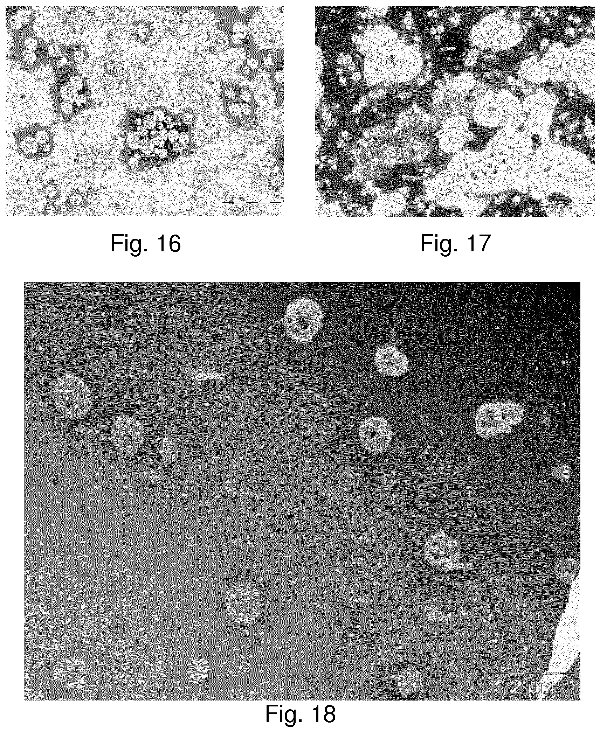

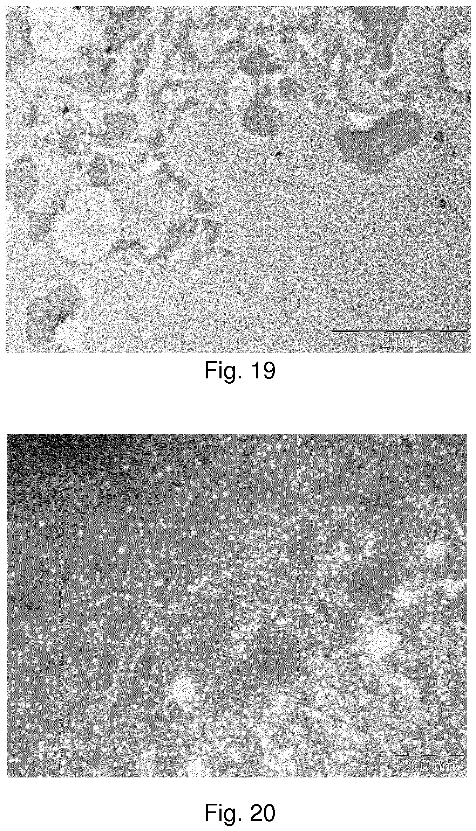

- A total of 100µL of GSH 10mM + 100µL of Example 19 filtered at 0.22 µm were incubated for 30 min at 37ºC. See Figs. 14 and 15. A partial degradation was observed. Areas with nanocapsules were still observed.
- A total of 100µL of GSH 10mM + 100µL of Example 19 filtered at 0.45 µm was incubated for 30 min at 37ºC. See Figs. 16 and 17. Partial or derisory degradation was observed.
- Two new incubations were carried out with glutathione. 100µL of GSH 10mM + 50µL of Example 19 (filtered at 0.22 µm) for 24h at 37ºC. It was repeated twice and in the two cases considerable sample degradation was observed. Aggregates sized larger than the filtration performed appeared due to the break in the capsular wall and the subsequent destabilising of its amphiphilic nature. See Figs. 18 and 19.
- A new incubation was carried out with glutathione. The test was conducted to conclude whether the capsules synthesised without disulphide in the wall also degraded in a reducer medium (glutathione). 100µL of GSH 10mM + 50µL of Example 18 (filtered at 0.22 µm) for 24h at 37ºC. Apparently no wall degradation due to glutathione was observed.

In all the degradation tests the characterisations were carried out by TEM.

### Degradation tests - Conclusions

- The 30 min treatment in GSH in Example 19 was not sufficient to degrade the wall.
- It was concluded that in Example 19 the polymeric capsular wall was functionalised with disulphide and this was degradable in the presence of a reducing medium in a concentration similar to that which can be found in the intracellular medium in tumour cells following incubation during 24h at 37ºC.
- It was also concluded that the nanocapsular wall not functionalised with disulphide in Example 19 without peptide was not degraded after an identical treatment to the one carried out in Example 19.

### Example 20 (reference example).

This Example is the same as Example 18 but incorporating a peptide in the nanocapsule wall.

Stage 1: The prepolymer was prepared as in Example 18.

A total of 5.98 g of Crodamol GTCC, 11.87 g of Ymer, 5.43 g of IPDI were loaded and heated to 85ºC in a N₂ atmosphere. The reaction was carried out during two and a half hours. Once the reaction had finished (valuing the NCO%) the reactor was cooled to 50 ºC and the contents was dissolved with 13.08 g of MEK. Then 1.64 g of Duomeen C dissolved in 25.05 g of MEK were added and it was left to react one more hour. The end of the reaction was checked by valuing the NCO level. The prepolymer was unloaded and kept in the fridge.

Stage 2. A tenth of the prepared prepolymer was taken and work continued with it.

In a suitable reactor, 6.25 g of prepolymer were loaded and using an addition funnel 25.64 g of water were added, dropwise. The process was carried out at room temperature with magnetic stirring. Once the emulsion was made, the peptide c-(RGDfK) previously dissolved in 5.31 g of water and neutralised with 100 µl of NaOH 1N was added. It was left to react about 10 minutes and 0.0618 g of DETA dissolved in 2.00 g of water were added. Finally the MEK evaporated.

A white, fluid liquid was obtained.

When it was left to rest the separation of the two phases was observed, leading to a solid, white layer and leaving the barely transparent liquid. When observing the product with the optical microscope imperfect capsules were seen with a great variety in size (1-15 µm). The capsules were filtered with 0.45 µm micro filters and using Light Scattering it was observed that the capsules were mainly sized 6 nm, and the most abundant populations were concentrated between 4 and 12 nm.

### Example 21 (reference example).

It began with preparing the prepolymer exactly as in the previous Example.

### Prepolymer:

A total of 5.99 g Crodamol GTCC, 11.68 g Ymer and 5.92 g IPDI were added, it was subjected to an inert N₂ atmosphere and heated to 85ºC and left to react for 2h 30min. When all the Ymer had finished reacting (valuation of excess NCO), it was cooled to 50ºC and the contents dissolved with 12.53 g of MEK. Then 1.65 g of Duomeen C dissolved in 27.29 g of dry MEK was added. A second NCO valuation was made to check the total NCO reaction.

### Incorporating the peptide:

A part weighting 6.2668 g was taken from the prepared polymer and the peptide solution c-(RGDfK) prepared with 0.054 g of it dissolved in 7.4168 g of DMSO (dimethylsulphoxide) and neutralised with 0.0104 g of triethylamine was added to it. After about 15 minutes 70 g of water were added and the emulsion was made. Then 0.034 g of DETA dissolved in 4.73 g of water were added. Finally the MEK was evaporated and the product was filtered.

White, fairly liquid emulsion. The capsules observed with the optical microscope were larger than in the previous Example, sized between 1 and 5 microns. Perfect, stable spheres were observed. No non-encapsulated oil or polymer remains were seen. The product was easily filtered with 0.450 µm micro filters.

The DLS results show that the emulsion is made up of two groups of capsules mainly (2 and 5 nm).

### Example 22 (reference example).

The same method was used as in Example 18 but replacing part of the MEK with acetone.

### Prepolymer:

Under an inert N₂ atmosphere and at 85 ºC, 11.6267 g of Ymer, 5.92 g Crodamol GTCC and 5.29 g IPDI were loaded. It was left to react for 2 hours. Once the reaction had finished (real NCO = 3.863% and theoretical NCO = 4.335%), the reactor was cooled to 50ºC and the contents dissolved with 15.32 g of MEK. Then 1.5950 g of Duomeen C dissolved in 16.70 g of MEK were added.

It was checked that all the Duomeen C had reacted, by conducting an NCO in excess valuation (real NCO = 0.030%, theoretical NCO = 0.883%). Finally, 12.00 g of acetone at a temperature below 15ºC were added. The product was unloaded and kept well-sealed in the fridge.

Various parts were taken from the prepolymer prepared and the following tests were conducted.

### Target:

A part weighing 6.8090 g was taken and put in the reactor under an inert atmosphere and at room temperature.

Under magnetic stirring, 25.13 g of water were added to make the emulsion. The water was added dropwise and the reactor was cooled with a mixture of water-ice. Then 0.0430 g DETA dissolved in 5.03 g of water were added. Finally the solvents evaporated and the product was filtered. A white, fluid emulsion was obtained and a separation of two phases was observed, one transparent liquid phase and a second solid phase floating on the surface.

With the microscope well defined capsules were observed sized smaller than a micron. Also capsule agglomerates were observed.

To one part weighing 6.8794 g of the prepolymer synthesised before, 0.054 g of the c-(RGDfK) peptide dissolved in 0.70 g of water and neutralised with 0.0280 g of triethylamine were added. The peptide action took place at temperatures below 15ºC and with magnetic stirring, and it was left to react for about 15 minutes. Once this time had elapsed, the product was emulsified with 26.44 g of water which were added gradually increasing the magnetic stirring. Then 0.0507 g DETA dissolved in 5.66 g of water at room temperature were added. Finally, the solvents evaporated and the product was filtered.

The product obtained was white and fluid, and unstable because it separated into two phases; one solid layer floating on the surface of the transparent liquid phase. This product was filtered using a 0.45 micron micro filter and the filtered transparent part was observed using SEM. Perfect, well defined and stable capsules were obtained when observed dry using SEM. This proved their structural stability in spite of their small size.

### Example 23 (reference example).

Example 18 was copied but incorporating the antitumoral agent, plitidepsin.

Under an N₂ atmosphere 11.5949 g Ymer, 6.03 g Crodamol GTCC and 6.21 g IPDI were loaded and the reaction was carried out at 75°C for 2 hours. The NCO was valued to check that all the Ymer had reacted (NCO_{theoretical} =5.624%, NCOᵣₑₐₗ =5.624%). The temperature was lowered to 50ºC and the mixture was dissolved with 14.08 g of MEK. Then 1.6431 g of Duomeen C dissolved in 25.08 g of MEK were added.

After an hour another NCO valuation was made to check the end of the reaction (NCO_{theoretical} =1.304%, NCOᵣₑₐₗ =0.770%). The reaction was stopped, the prepolymer was unloaded and kept well-sealed in the fridge.

### Encapsulation of Plitidepsin

A part weighing 6.9108 g was taken from the prepolymer prepared and placed in a suitable reactor avoiding, as much as possible, any air entering by passing a current of nitrogen. Then, carefully, 0.0108 g of plitidepsin (drug) was added with magnetic stirring. Once the drug was dissolved the emulsion was made with 20.68 g of water added slowly using an addition funnel. Finally 0.0303 g DETA dissolved in 5.02 g of water were added and left under stirring for approximately one hour.

A white emulsion was obtained that separated into two phases. Under the optical microscope well rounded, perfect capsules were observed sized between 0.5 and 2 microns.

After filtering the emulsion was transparent and stable, and it did not separate into different phases.

Using SEM, nanocapsules were observed with an average size of between 6 and 10 nm. Using DLS two size population distributions were detected, one average size population around 13 nm and another average size of 350 nm.

### Example 24 (reference example).

This was conducted in the same way as Example 13 reducing the amount of the reagents and diluting the solids to half.

A total of 0.5056 g Duomeen C were dissolved in 75.31 g of water. Another solution was prepared separately by mixing 1.7825 g of Ymer, 0.9470 g of Crodamol GTCC, 0.82 g of IPDI and to fluidify this mixture 2 g approximately of acetone were added. At room temperature the second mixture was added to the first and stirred with the Ultra-Turrax at 15,000 rpm for 30 min.

The emulsion obtained was stable and consisted on a white, fluid liquid. The capsules observed with the optical microscope were well-defined and stable and sized between 0.5 and 1.5 microns.

After the filtration and drying process nanocapsules with a diameter of about 150 nm could be observed.

### Example 25 (reference example).

This product was synthesised as in Example 24 above replacing part of the Duomeen C with c-(RGDfK) peptide and Triameen C to compensate the functionality.

A total of 0.9340 g of Crodamol GTCC, 1.7699 g Ymer and 0.83 g IPDI were mixed. The mixture was diluted with 2 g of acetone and another mixture prepared by dissolving 0.4192 g Duomeen C, 0.0808 g peptide and 0.0330 g of Triameen C in 75.12 g of water was added. It was shaken at 15,000 rpm for 30 minutes at room temperature.

A white, stable emulsion was obtained. The capsules observed with the optical microscope were sized between 0.5 and 1 micron

### Example 26 (reference example).

It was synthesised the same way as the product in Example 23 replacing part of the oil, Crodamol GTCC, with the drug.

A total of 1.7696 g Ymer were mixed with 0.9043 g Crodamol GTCC and 0.0229 g of drug (plitidepsin). This mixture was fluidified with 2 g de acetone.

Separately 0.4871 g Duomeen C were dissolved in 75.65 g of water and the previously prepared mixture was added to it. It was left to react half an hour under the same temperature and stirring conditions.

The emulsion had the same appearance as the ones in the last two Examples above and the capsules were sized smaller than a micron.

### Example 27 (reference example).

In this Example the two previous Examples were combined, based on the same philosophy as in Example 23. In other words the same methodology as in Example 23 was followed, but replacing part of the oil and part of the Duomeen C with the corresponding components.

A total of 0.9085 g of Crodamol GTCC, 1.7744 g Ymer, 0.8109 g IPDI and 0.0250 g of drug (plitidepsin) were mixed. The mixture obtained was dissolved with 2.5 g of acetone. Separately 0.081 g of c-(RGDfK) peptide, 0.4186 g Duomeen C and 0.0491 g Triameen C were dissolved in 75.26 g of water. The organic mixture was added to the aqueous mixture and it was left to react for 30 minutes with stirring at 15,000 rpm.

A good, stable white emulsion was obtained and capsules sized smaller than a micron were observed.

### Example 28.

This Example introduces a second hydrophobic prepolymer for increasing the encapsulation power, and the strength of the particles.

A total of 6.7632 g of IPDI, 5 g of Crodamol GTCC and 520 µl (0.655 g) of 2,2-dithiodiethanol (DEDS), previously stirred in vortex, were added simultaneously to a reactor at 50ºC. The remains were entrained with diethyl ether. It was observed that the 2,2-dithiodiethanol was more dense and appropriate stirring was required to ensure that no phases were formed during the reaction. When the reaction started, the reaction medium turned cloudy.

One hour later 7.5 g of dry YMER N-120 were added, and entrained with 1 ml of dry acetone. The dry acetone can be replaced preferably with diethyl ether, which always has a smaller water content and evaporates more quickly in the reaction medium.

When the theoretical NCO was reached (approximately after 4 to 5 hours reaction time) the heating bath was removed and 3.75 g of LAP 100D solubilised in 8g of dry MEK were added.

After 45 min the reactor was cooled to about 5-10ºC and 1.9425 g of IPDI and 1.8075 g of Bayhydur 3100 in 16 ml of dry MEK were added. These two isocyanates are the second prepolymer, which will form the second, internal layer of the wall. A distinctive thickening of the sample was observed. Two drops of BYK-028 antifoaming agent (at the most there can be 40g of MEK overall) were added.

Before emulsifying, the NCO presence in the reaction was checked using IR.

Then, dropwise 260 ml of milliQ water basified to a pH between 11 and 12 and cooled to a temperature between 5 and 10 ºC, were added.

When the phase inversion took place (fairly viscose) 1024 µl (1.1461 g) of DETA were added in the compensated pressure funnel while continuing to add, dropwise, the rest of the water with the DETA at greater speed.

The stirring and the cooling to 10ºC were maintained until the NCO signal disappeared from the IR spectrum.

An even, white-bluish product was obtained. The MEK was removed in the rotary evaporator. The pH was adjusted to 7 with diluted HCI and it was diluted to 10% solids.

A stable emulsion was obtained that was stored in the fridge. It was diluted to 1% for observation under the transmission electronic microscope. It lyophilised easily, by freezing it with liquid nitrogen, and obtaining a white powder. It was redispered in deionised water or in PBS without any problem, stirring for 3 min. Sometimes it is advisable to leave it to rest for 24 hour so that the emulsion is fully reconstituted.

This Example is a particularly advantageous embodiment of the invention.

Fig. 22 shows the TEM image of the particles obtained.

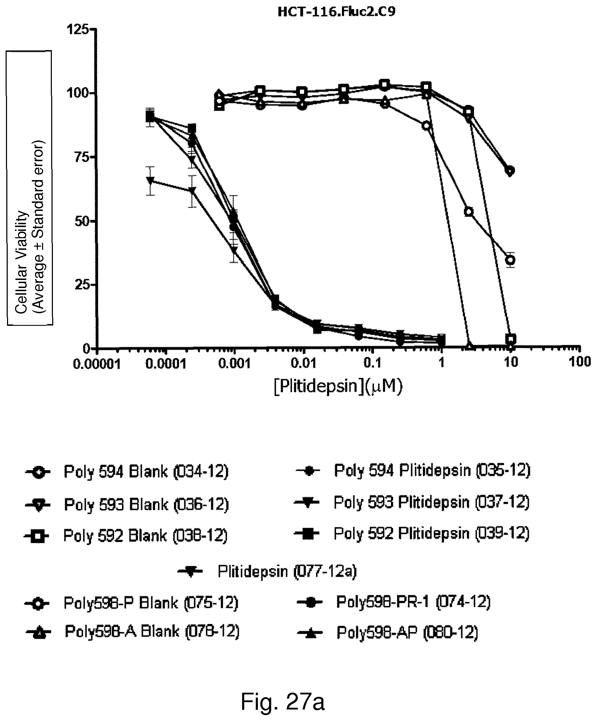

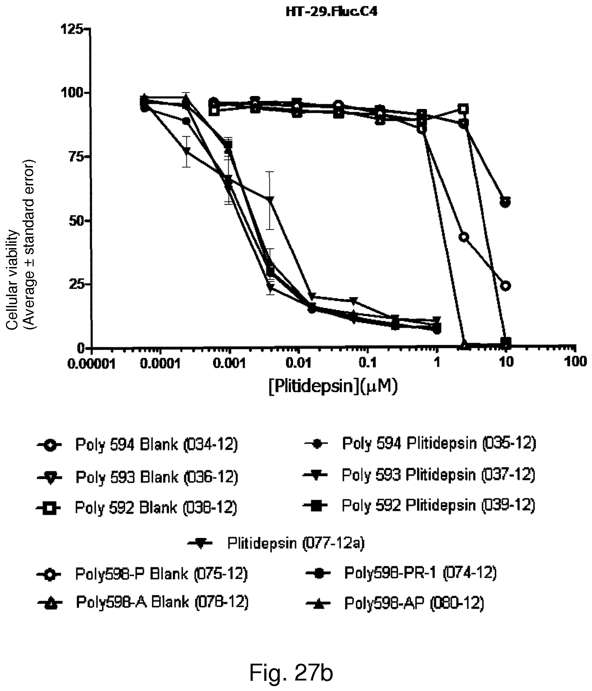

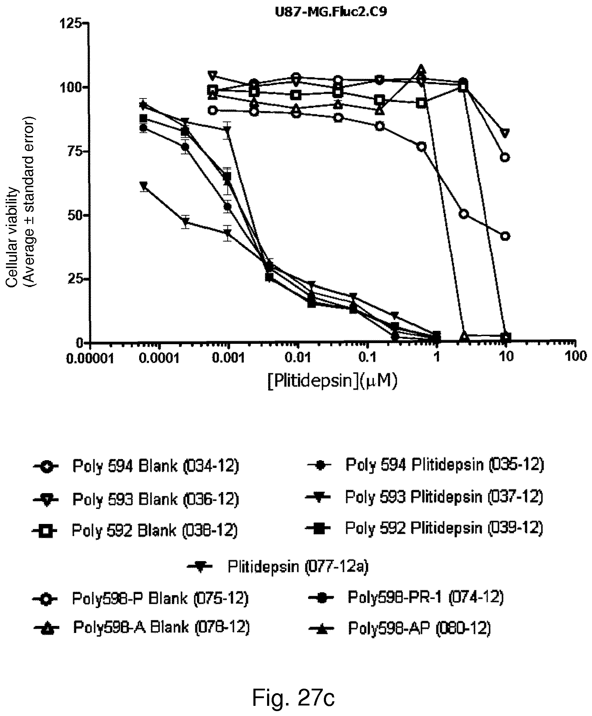

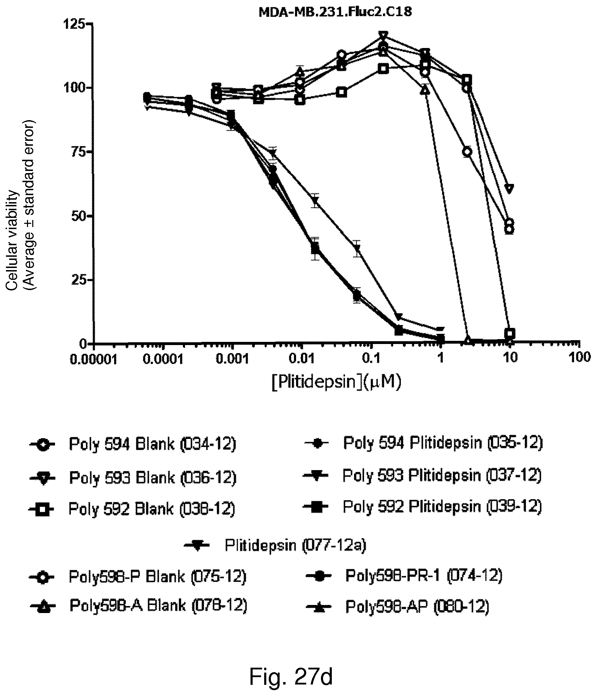

Figs. 27a to 27d show a cytotoxicity study on nanocapsules concerning Example 28 by MTT in cells with an overexpression of lucipherase (HCT-116.Fluc2.C9, HT-29.Fluc.C4, U87-MG.Fluc2.C9 and MDA-MB.231.Fluc2.C18), after 72 hours incubation with nanocapsules in antibiotic-free culture media (RPMI).

The maximum concentration tested was 10 µM of plitidepsin equivalent for the empty nanocapsules, and 1 µM of plitidepsin for the loaded nanocapsules, with 8 serial quarter log dilutions. For each concentration, in each assay, 6 replicas were tested. The assays were repeated at least 3 times.

The empty nanocapsules were not toxic at the concentration for therapeutic use.

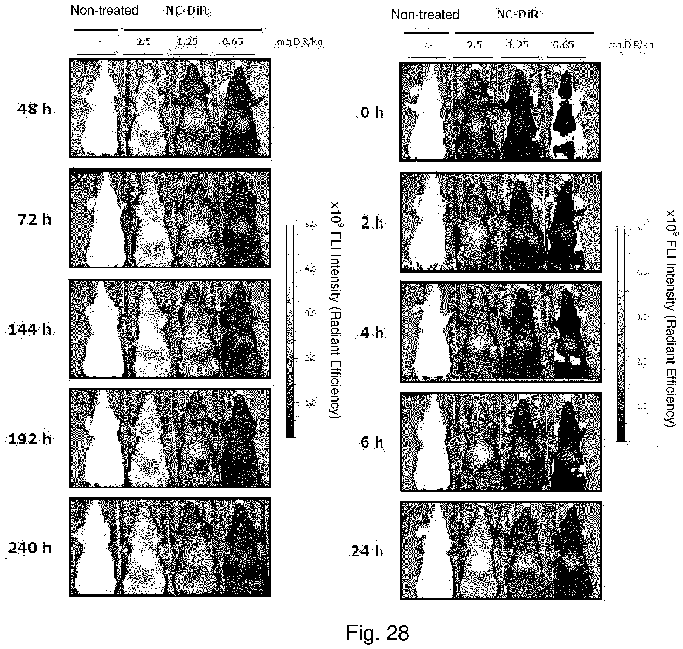

Fig. 28 shows a biodistribution study on athymic mice without a tumour in the nanocapsules in Example 28 loaded with DiR.

The emulsions administered have been given maximum solubility and they were as follows:
- NC-DiR_0.5 mg DiR/mL (20% solids)_2.5 mg DiR/kg_n (number of mice used)=4
- NC-DiR_0.25 mg DiR/mL (20% solids)_1.25 mg DiR/kg_n=3
- NC-DiR_0.13 mg DiR/mL (20% solids)_0.65 mg DiR/kg_n=4

All the mice survived the injections showing a prolonged photoluminescence.

### Example 29.

This Example is the same as Example 28 above, but instead of using LAP 100D, Priamine 1074 was used.

A total of 4.5506 g of IPDI, 5 g of Crodamol GTCC and 0.6039 g of 2,2-dithiodiethanol (DEDS), previously stirred in vortex agitator, were added simultaneously to a reactor at 50ºC. The remains were entrained with diethyl ether. It was observed that the 2,2-dithiodiethanol was more dense and appropriate stirring was required to ensure phases did not form during the reaction. When the reaction started, the medium turned cloudy.

An hour later 6.91 g of dry YMER N-120 were added, and entrained with 1ml of dry acetone.

When the theoretical NCO was reached (approximately after 4 hours reaction time) the heating bath was removed and 1.5625 g of Priamine 1074 solubilised in 8 g of dry MEK were added and it was activated with 2 drops of triethylamine.

After 45 min the reactor was cooled to about 5-10ºC and 1.77 g of IPDI and 1.25 g of Bayhydur 3100 in 16 ml of dry MEK were added. These two isocyanates were the second prepolymer, which will form the second, internal layer of the wall. A distinctive thickening of the sample was observed. Two drops of BYK-028 antifoaming agent (at the most there can be 40 g of MEK in total) were added.

Before emulsifying the NCO presence in the reaction was checked using IR.
Then, dropwise 260 ml of milliQ water basified to a pH between 11 and 12 and cooled to a temperature between 5 and 10 ºC, were added.

When the phase inversion took place (fairly viscose) 1237 µl (1.1778 g) of DETA were added in the compensated pressure funnel while continuing to add, dropwise, the rest of the water with the DETA at greater speed.

The stirring and the cooling to 10ºC were maintained until the NCO signal disappeared from the IR spectrum.

An even, white-bluish product was obtained. The MEK was removed in the rotary evaporator. The pH was adjusted to 7 with diluted HCI and it was diluted to 10% solids.

A stable emulsion was obtained that was stored in the fridge. It was diluted to 1% for observation under the transmission electronic microscope. It lyophilised easily, by freezing it with liquid nitrogen, and obtaining a white powder. It was redispered in deionised water or in PBS without any problem, stirring for 3 min. Sometimes it is advisable to leave it to rest for 24 hour so that the emulsion is fully reconstituted.

This Example is a particularly advantageous embodiment of the invention.

### Example 30.

This Example is a variation of Example 29 above, in that it is cross-linked with cystamine hydrochloride instead of with DETA.

A total of 4.5506 g of IPDI, 5 g of Crodamol GTCC and 0.6039 g of 2,2-dithiodiethanol (DEDS), previously stirred in vortex agitator, were added simultaneously to a reactor at 50ºC. The remains were entrained with diethyl ether. It was observed that the 2,2-dithiodiethanol was more dense and appropriate stirring was required to ensure phases did not form during the reaction. When the reaction started, the medium turned cloudy.

An hour later 6.91 g of dry YMER N-120 were added, and entrained with 1ml of dry acetone.

When the theoretical NCO was reached (approximately after 4 hours reaction time) the heating bath was removed and 1.5625 g of Priamine 1074 solubilised in 8 g of dry MEK were added and it was activated with 2 drops of triethylamine.

After 45 min the reactor was cooled to about 5-10ºC and 1.77 g of IPDI and 1.25 g of Bayhydur 3100 in 16 ml of dry MEK were added. These two isocyanates were the second prepolymer, which will form the second, internal layer of the wall. A distinctive thickening of the sample was observed. Two drops of BYK-028 antifoaming agent (at the most there can be 40 g of MEK in total) were added.

Before emulsifying the NCO presence in the reaction was checked using IR.

Then, dropwise 260 ml of milliQ water basified to a pH between 11 and 12 and cooled to a temperature between 5 and 10 ºC, were added.

When the phase inversion took place (fairly viscose) 3,825 g of cystamine hydrochloride previously dissolved in as little an amount as possible of milliQ water basified to pH 11 were added, while continuing to add, dropwise, the rest of the water with the cystamine hydrochloride at greater speed.

The stirring and the cooling to 10ºC were maintained until the NCO signal disappeared from the IR spectrum.

An even, white-bluish product was obtained. The MEK was removed in the rotary evaporator. The pH was adjusted to 7 with diluted HCI and it was diluted to 10% solids.

A stable emulsion was obtained that was stored in the fridge. It was diluted to 1% for observation under the transmission electronic microscope. It lyophilised easily, by freezing it with liquid nitrogen, and obtaining a white powder. It was redispered in deionised water or in PBS without any problem, stirring for 3 min. Sometimes it is advisable to leave it to rest for 24 hour so that the emulsion is fully reconstituted.

Adding the cystamine hydrochloride in a base medium produces a certain degradation of the cystamine hydrochloride, therefore it loses part of its effectiveness. Therefore, one alternative is to add the cystamine hydrochloride in an acid medium, in which case a longer waiting period is required (a few days) until the NCO signal disappears from the IR spectrum. This is also applicable in Example 35 below.

### Example 31. Method of Encapsulating plitidepsin

Using a prepolymer formed at 50ºC with IPDI, DEDS, Crodamol GTCC and YMER N-120 (see, for example, Examples 28, 29 and 30) the bath heating was switched off and the necessary amount of LAP 100D in 10 g of MEK was added. It was left to react for 45 min and the formation of polyurea was controlled using IR.

Next the reactor was cooled to 10ºC and the rest of the MEK and the excess isocyanate was added. The reaction medium was homogenised and the proportional part of the prepolymer + MEK was taken. This proportional part of prepolymer was added to the amount of plitidepsin needed to be at 1% with respect to the prepolymer + the cross-linking amine (DETA) (the amount of MEK is not taken into consideration for this calculation). This way, when it is emulsified so that it is at 10% solids, it will have 0.01% of plitidepsin.

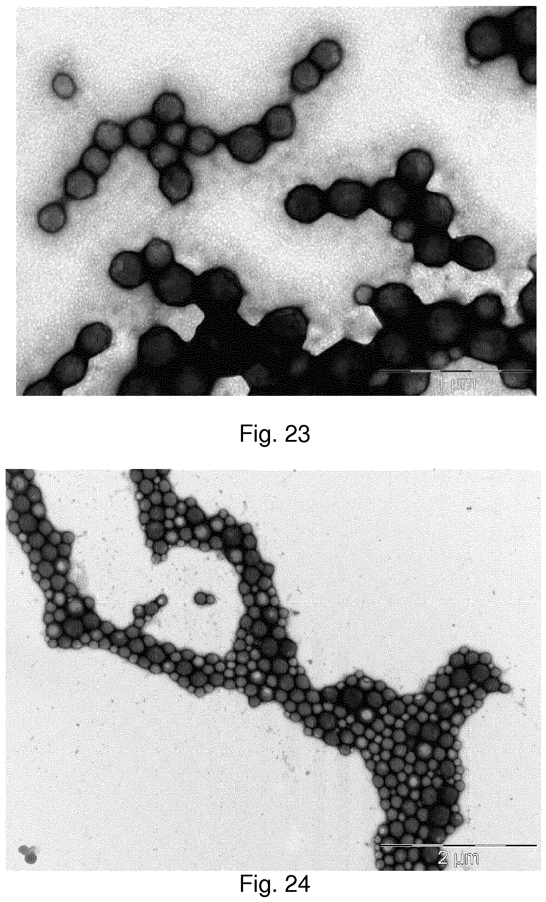

Fig. 23 shows the TEM image of the particles obtained.

### Example 32. Method of derivatization of a peptide that promotes the nanocapsule wall (reference example).

A total of 122 mg of Bayhydur 3100 were mixed with 36.3 mg of c-(RGDfK) dissolved in 0.9 ml of milliQ water at 5ºC and basified to a pH 11.5. It was left to react for 90 minutes, stirring in a vortex agitator every 10-15 minutes. The reaction was controlled by HPLC, after 90 min the peptide derivatization performance was 95%.

A prepolymer was emulsified (see, for example, the prepolymer in Example 28) + MEK + plitidepsin adding, dropwise, the already reacted aqueous mixture of Bayhydur 3100 with the c-(RGDfK). Before starting the emulsion, it was checked using IR, that there were still a large amount of free isocyanates. If the inversion phase had not taken place, more water would have been added until the inversion phase occurred. Once the inversion phase had taken place the rest of the cold, basified water was added together with the second compound (the cross-linking amine).

The MEK was removed in a rotary evaporator, leaving the product free of organic solvents and suitable for being lyophilised and then redispersed in PBS.

### Example 33. Method of encapsulating hydrophobic fluorophore (reference example).

In a previously weighed (tarred) 100 ml round bottomed flask the prepolymer + MEK and plitidepsin, were added, where appropriate. A total of 5 mg of DIR (1,1'-dioctadecyl-3,3,3',3'-tetramethylindotricarbocyanine iodide) dissolved in the minimum amount of dry acetone were added. The mixture was stirred and was ready for being emulsified following, for example, the method in Example 28.

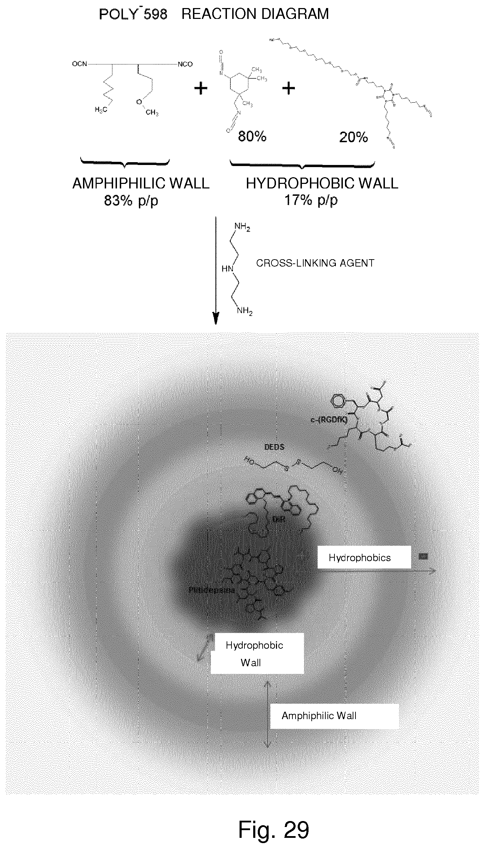

Fig. 29 shows a reaction diagram and a theoretical representation of the ideal structure of the nanocapsule in Example 28 loaded and functionalised following Examples 31-33.

### Example 34 (reference example).

This Example synthesises a variant of Example 28 replacing DEDS with 1,6-hexanediol. This way a similar product is obtained without disulphide in the membrane.

A total of 6.7124 g of IPDI, 6.6363 g of Crodamol GTCC and 0.4765 g of 1,6-hexanediol were added, simultaneously to a reactor at 55-60ºC under an inert gas current and it was left to react with stirring at 265 rpm for one hour. After one hour 10.2568 g of dry YMER N-120 were added.

When the theoretical NCO is reached (approximately after 4 hours reaction time) the heating batch was removed 3.0670 g of LAP 100D solubilised in 16,7756 g of dry MEK were added.

One hour later the reactor was cooled to about 5-10ºC and 2,0836 g of IPDI and 1.8408 g of Bayhydur 3100 in 23 ml of dry MEK were added. These two isocyanates are the second prepolymer, which will reinforce the wall forming a second, internal layer.

Before emulsifying the NCO presence in the reaction was checked using IR.

Then, dropwise 250 ml de milliQ water basified to a pH between 11 and 12 and cooled to a temperature between 5 and 10 ºC, were added. It was stirred up to 400 rpm.

When the phase inversion took place 1100 µl (1,1589 g) of DETA was added in the compressed pressure funnel while continuing to add, dropwise, the rest of the water with DETA.

The stirring and the cooling to 10ºC were maintained until the NCO signal disappeared from the IR spectrum.

An even, white, bluish product was obtained. The MEK evaporated. The pH was adjusted to 7 with dilute HCI and it was diluted to 10% solids.

A stable emulsion was obtained which was stored in the fridge. Using electronic microscopy (SEM) agglomerated capsules were observed with diameters smaller than 200 nm.

### Example 35.

A variant of Example 28 where a second very hydrophobic prepolymer was introduced, which will form an internal wall with greater encapsulation power for very hydrophobic molecules. However it was cross-linked with cystamine hydrochloride instead of DETA so that the wall was highly degradable in a medium rich in Glutathione (GSH).

A total of 6.7632 g of IPDI, 5 g of Crodamol GTCC, 520 µl (0.655g) of 2,2-dithiodiethanol (DEDS) and 8 g of YMER N-120, previously stirred in a cortex agitator, were added simultaneously to a reactor at 50ºC. The remains were entrained with diethyl ether. The stirring had to be vigorous so that phases did not form during the reaction. When the reaction started, the reaction medium turned cloudy.

Once the theoretical NCO was reached (approximately after 4 hours reaction time) the heating bath was removed and 3.6265 g of LAP 100D solubilised in 8 g of dry MEK were added.

At the same time, in a 100 mL round-bottom flask, 1.9425 g of IPDI and 1.8075 g of Bayhydur 3100 dissolved in 20 ml of dry MEK were added. Next, in an inert atmosphere, dropwise, 3.34 g of Priamine dissolved in 8 mL of MEK/acetone were added. The reaction was followed using FT-IR.

After 45 min the reactor was cooled to 5-10ºC and the hydrophobic prepolymer of IPDI-PRIAMINE-B3100 dissolved in MEK/ACETONE were added. These two isocyanates and the Priamine were the second prepolymer, which will reinforce the wall forming a very hydrophobic second, internal layer. Two drops of BYK-028 antifoaming agent were added.

Before emulsifying the NCO presence in the reaction was checked using FT-IR.

Then, dropwise, 270 ml de milliQ water basified to a pH between 11 and 12 and cooled to a temperature between 5 and 10 ºC.

When the phase inversion took place 2,343 g of cystamine hydrochloride basified with triethylamine were added in the compressed pressure funnel while continuing to add, dropwise, the rest of the water with cystamine hydrochloride at greater speed.

The stirring and the cooling to 10ºC were maintained until the NCO signal disappeared from the IR spectrum.

An even, white, bluish product was obtained. Solvents were evaporated in the rotary evaporator. The pH was adjusted to 7 with dilute HCI and it was diluted to 10% solids.

A stable emulsion was obtained that was stored in the fridge. It was diluted to 1% for observation under the transmission electronic microscope. It lyophilised easily, by freezing it with liquid nitrogen, and obtaining a white powder. It was redispered in an 80:20 mixture of water/acetone stirring for 3 min. Sometimes it is advisable to leave it to rest for 24 hour so that the emulsion is fully reconstituted.

Fig. 24 shows the TEM image of the particles obtained.

### Example 36. NA-834 Prepolymer - Encapsulation of the Miami Vice® Fragrance (reference example)

### Method for synthesising the NA-834 prepolymer

In a N₂ current, 49.2402 g of YMER N-120 and 30.0951 g IPDI (NCO:8/OH:2.5) were added. The reaction was carried out at 55-60ºC with mechanical stirring at 260 rpm. After 3-3.5 hours, the total YMER N-120 reaction was checked by valuing the NCO level (NCO_{theoretical}= 8.949, NCOᵣₑₐₗ= 8.945). Then the Duomeen T dissolved in MEK was loaded. The end of the reaction was checked using IR0 follow-up.

### Fragrance encapsulating method

A total of 32.3615 g of the previously synthesised NA-834 prepolymer were taken, and placed in a reactor with a N₂ atmosphere. The mixture was fluidified by adding, approximately, 9 g of MEK and stirring mechanically. A total of 24.5280 g of the fragrance were added and then 4.6980 g of polymeric MDI were added, which will lead to the formation of the interior wall. Once the mixture was homogenised, the emulsion was formed by adding, dropwise, 70.0383 g of cold water (from the fridge, 4ºC). The emulsion was carried out by cooling the reactor with a water-ice mixture bath (0-5ºC). Finally, to the water remaining in the addition funnel (approximately 10 g), 1490 µl of diethylenetriamine (DETA) were added, the amine stechiometric equivalents for finishing the reaction with the isocyanate still in excess. The reactions were controlled using IRs before and after adding each reagent.

### Characterisation

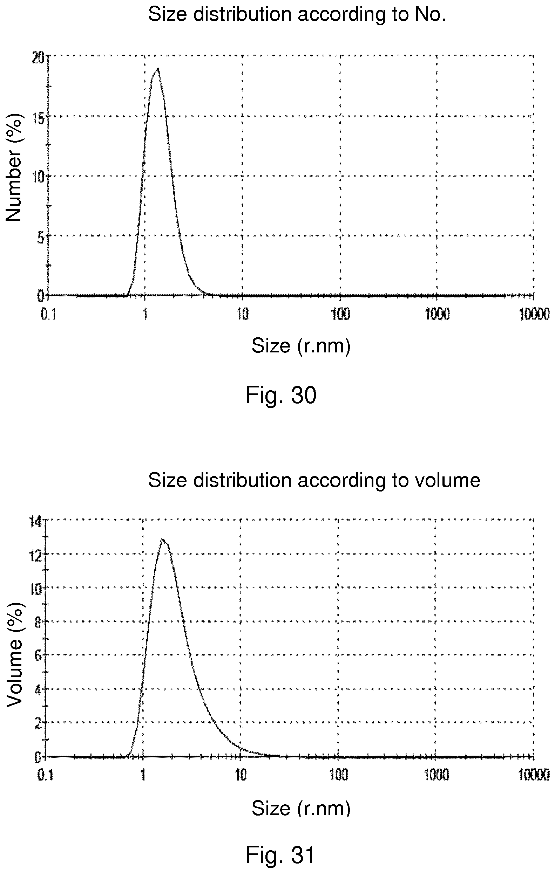

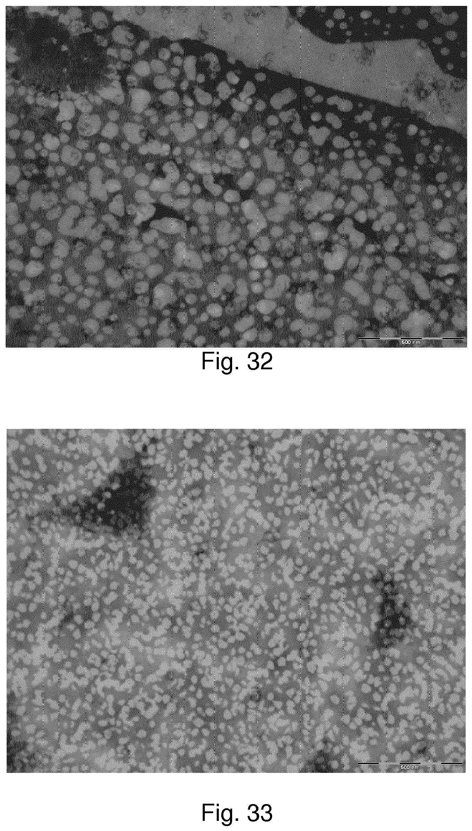

Figs. 30 and 31 show a graphical representation of the particle size distribution by DLS (in Fig. 30 the ordinate axis indicates the % of units, and in Fig. 31 it indicates the % volume). Using TEM a defined main population of nanoparticles was observed, with a diameter of 10-30 nm (Figs. 32 and 33: images by transmission electron microscopy (TEM) of fragrance nanocapsules at 20%.

To study the so-called long-lasting effect of the fragrance nanoparticles, three different product solutions were prepared:
A) control solution, made up of free fragrance (3%) in a mixture of water (22%) and ethanol (75%).
B) fragrance nanocapsules (3%), in aqueous medium (water+polymer (97%).
C) fragrance nanocapsules (3%), in water (water+ polymer 22%) and ethanol (75%).

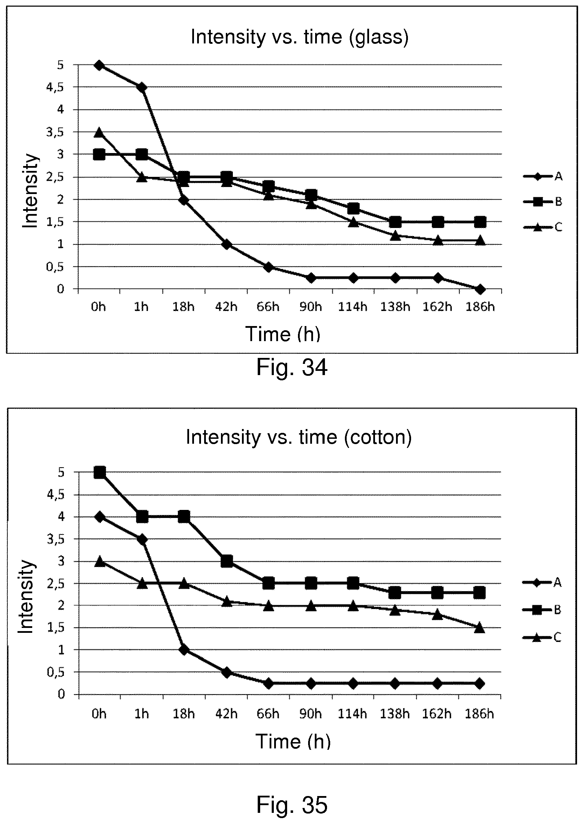

The three prepared solutions were applied to two types of material; glass and cotton. On this basis, the evolution of the odour intensity over time was studied. Figs. 34 and 35 show the results, which were assessed aromatically, and independently, by 5 individuals.

This Example is a particularly advantageous embodiment of the invention.

### Example 37. Poly 620

This Example cross-links with L-lysine, i.e.: the second compound is L-lysine. The size of the nanocapsules was reduced considerably, obtaining nanocapsules with a size of 50 nm. As the L-lysine contains carboxylic groups, the nanocapsule's external hydrophilic nature increases. Its formula is:

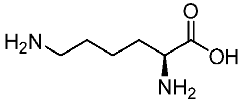

In a reactor duly equipped with mechanical stirring at 50ºC and with an inert atmosphere 3.3814 g of IPDI, 0.75 g of Crodamol GTCC, 0.15 g of 2,2-dithiodiethanol (DEDS), 5.5 g of YMER N-120 and 10 mg of DBTL were added.

When the theoretical NCO was reached (approximately after 4-5 reaction time) the heating bath was removed and 1.375 g of TAP 100D solubilised in 8 g of dry THF were added. The stirring was maintained and it was left to react for 30 min. The TAP 100D is more hydrophobic than LAP 100D, so that with a smaller amount the same hydrophobic nature was obtained. This way, the superficial hydrophilic nature could be increased by adding YMER N120 and L-lysine if required to compensate the TAP 100D that was not added. Its formula is

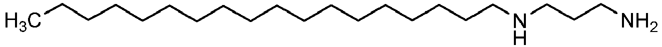

The reactor was cooled to about 5 to 10ºC and 0.485 g of IPDI and 0.904 g of Bayhydur 3100 in 16 ml of dry MEK were added on top of the amphiphilic prepolymer (AMPHIL). As already mentioned, the Bayhydur 3100 can behave as a linker (linking element) between the cRGDfK peptide (in the event it is desirable to include a peptide to convey it to the tumour cells) and the nanocapsule. The free IPDI will be the internal hydrophobic prepolymer which will increase the barrier effect.

Then, dropwise, 140 mL of milliQ water basified to a pH between 11 and 12 cooled to a temperature between 5 and 10 ºC, was added.

After the phase inversion 1,110 g of L-lysine were added in the compensated pressure funnel while continuing to add, dropwise, the rest of the water with the L-lysine solution at greater speed.

After 20 min, using IR the formation of urea functional groups was observed. The stirring was maintained and the cooling to 5 to 10ºC until the NCO signal disappeared from the IR spectrum.

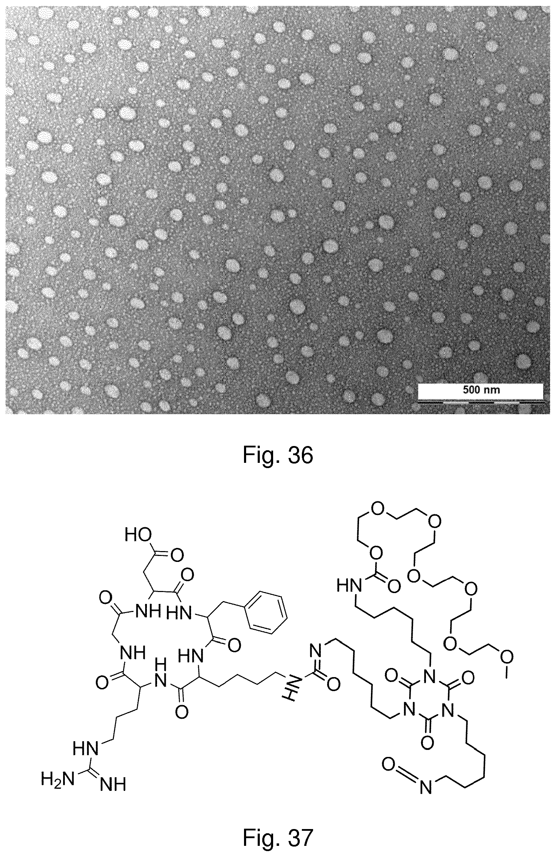

Fig. 36 shows a TEM image of the nanoparticles obtained.

This Example is a particularly advantageous embodiment of the invention.

### Example 38. Poly 636 (AMPHIL-HYFOB)

This Example introduced a second, hydrophobic prepolymer which, unlike previous Examples, also contained hydrophobic side chains and disulphide bridges in its main chain to increase the encapsulating power, but without losing degradability in the face of glutathione.

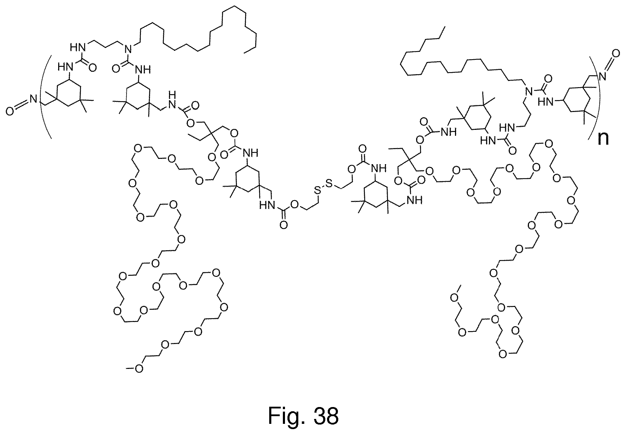

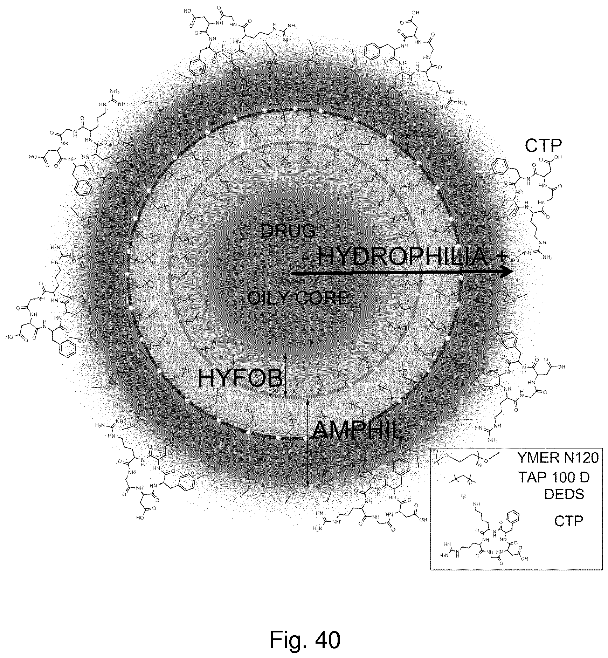

In a reactor duly equipped with mechanical stirring at 50ºC and with an inert atmosphere 3.3814 g of IPDI, 0.75 g of Crodamol GTCC, 0.15 g of 2,2-dithiodiethanol (DEDS), 5.5 g of YMER N-120 and 10 mg of DBTL were added. When the theoretical NCO was reached (approximately after 4 to 5 hours reaction time) the heating bath was removed and 1.45 g of TAP 100D solubilised in 8 g of dry THF were added. The stirring was maintained and it was left to react for 30 min. This amphiphilic prepolymer is called AMPHIL in Figs. 38 and 40.

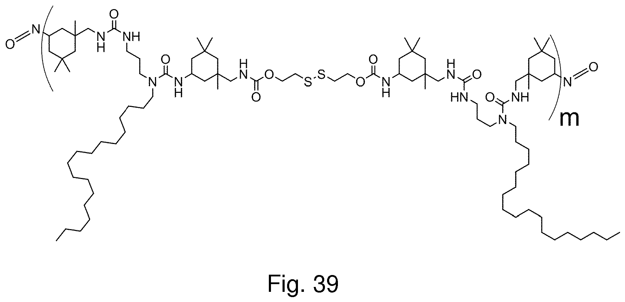

At the same time, in a 25 mL Schlenk at 50ºC purged with nitrogen, 0.486 g of IPDI, 0.15 g of DEDS and 10 mg of DBTL in 2 mL of dry THF were added. After 1 hour reaction time, 0.15 g of TAP 100D in 4 mL of dry THF was added and the heating was switched off. The stirring was maintained and it was left to react for 30 min. This hydrophobic prepolymer is called HYFOB in Figs. 39 and 40.

The reactor was cooled to about 5 to 10ºC and the hydrophobic prepolymer (HYFOB) and 0.904 g of Bayhydur 3100 in 16 ml of dry MEK was added on to the amphiphilic compound (AMPHIL). Bayhydur 3100 behaved as a linker (linking element) between the cRGDfK peptide and the nanocapsule (see Fig. 37). HYFOB was the hydrophobic prepolymer with disulphide bridges in the wall to make it degradable in the face of glutathione.

Then, dropwise, 140 mL de milliQ water basified to a pH between 11 and 12 cooled to a temperature between 5 and 10 ºC, was added.

After the inversion phase, 0.500 g of L-lysine were added in the compensated pressure funnel while continuing to add, dropwise, the rest of the water with the L-lysine solution at greater speed.

After 20 min, using IR the formation of urea functional groups was observed.

Next 0.236 g of DETA were added. The stirring and the cooling to 5 to 10ºC were maintained until the NCO signal disappeared from the IR spectrum.

Fig. 40 shows a theoretical representation of the structure of the microcapsule in this Example.

Result: A sample of 0.2 mL 10 mg/mL previously filtered with a 220 nm filter, was mixed with 0.4 mL of GSH 10 mM at 37 ºC. Stirring continued at 37ºC and the degradation of 100 nm nanocapsules continued and consequently the formation of aggregates larger than 1 micron.

It was proved that adding disulphide groups to the HYFOB accelerated the degradation of the nanocapsules in a reducing medium similar to the one found in the cytosol of cancerous cells.

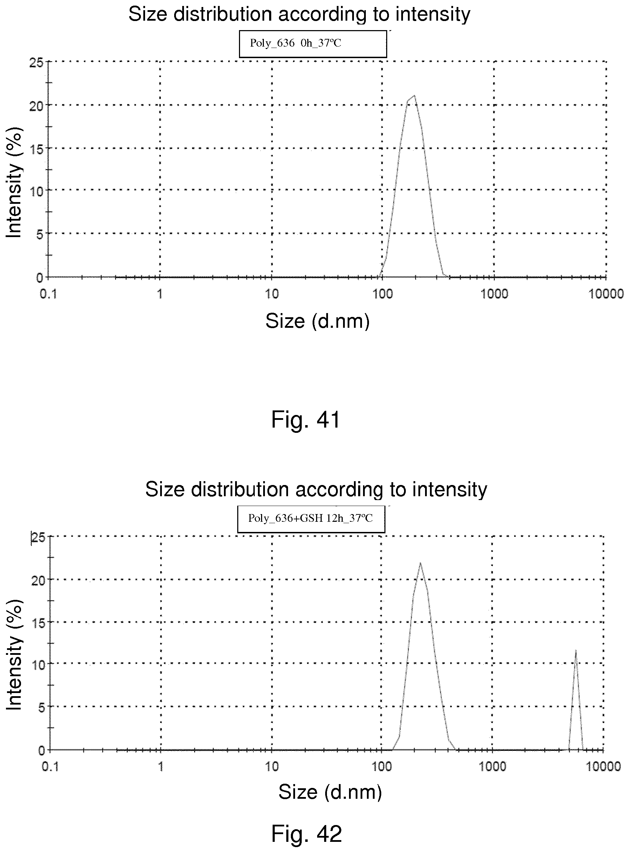

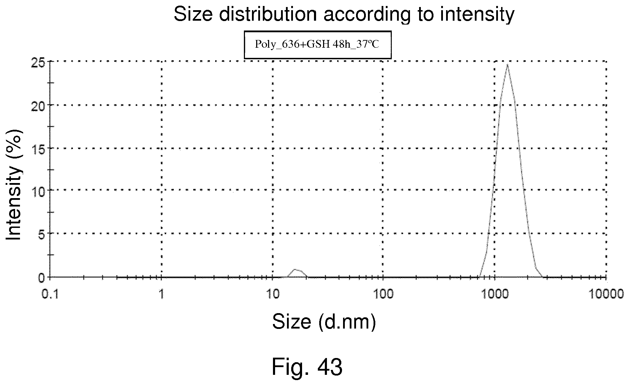

Figs. 41 to 43 represent the evolution of the degradation of microcapsules in this Example due to the reducing action of glutathione (GSH) according to the time period. Fig. 41 shows the initial state, Fig. 42 is after 12 hours, and Fig. 43 is after 48 hours

This Example is a particularly advantageous embodiment of the invention.

### Example 39. Coating biocompatible metals- Polytitanium

This Example describes a new application of the microcapsules according to the invention. It consists of the surface coating of a biocompatible material, preferably a biocompatible metal and very preferably titanium or its alloys, with microcapsules according to the invention.

In this Example the cross-linking is done with EDA. Microcapsules are functionalised on top of a titanium alloy (Ti₆Al₄V), which is a suitable biocompatible alloy, for example, for producing dental implants.

In a reactor duly equipped with mechanical stirring at 50ºC and with an inert atmosphere 3.3814 g of IPDI, 0.75 g of Crodamol GTCC, 0.15 g of 2,2-dithiodiethanol (DEDS), 5.5 g of YMER N-120 and 10 mg of DBTL were added. When the theoretical NCO was reached (approximately after 4 to 5 hours reaction time) the heating bath was removed and 1.375 g of TAP 100D solubilised in 8 g of dry THF were added. The stirring was maintained and it was left to react for 30 min. The reactor was cooled to about 5 to 10ºC and 0.485 g of IPDI and 0.904 g of Bayhydur 3100 in 16 ml of dry MEK were added on to the amphiphilic prepolymer (AMPHIL). The hydrophobic prepolymer (HYFOB) and 0.904 g of Bayhydur 3100 in 16 ml of dry MEK were added on to the amphiphilic compound (AMPHIL).

Then, dropwise, 140 mL de milliQ water basified to a pH between 11 and 12 cooled to a temperature between 5 and 10 ºC, was added.

After the inversion phase, 50 % mass of the total EDA (ethylenediamine) to be added, was added. After 15 min, using IR a reduction in the isocyanate group signal was observed.

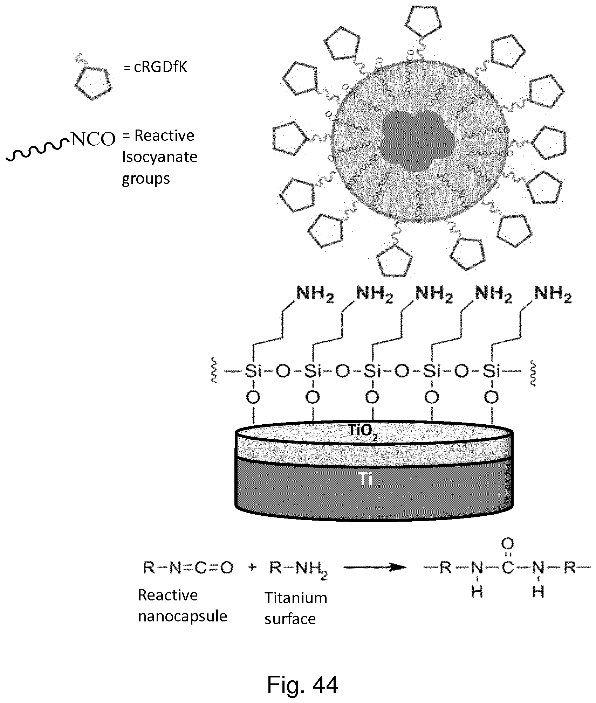

Next, titanium discs (particularly, from the (Ti₆Al₄V) alloy, like the ones shown in Fig. 44), were immersed in the emulsion containing the partially cross-linked nanocapsules for 1 hour at 5 to 10ºC. The discs of titanium underwent a superficial activation process thanks to which they had covalently linked amines on their surface. The microcapsules are reactive with the superficial amines on the titanium, in this way they react covalently with them and remain chemically attached to the surface of the metal.

After 1 hour the remaining 50 % of EDA was added. The stirring was maintained and the cooling to 5 to 10ºC until the NCO signal disappeared from the IR spectrum.

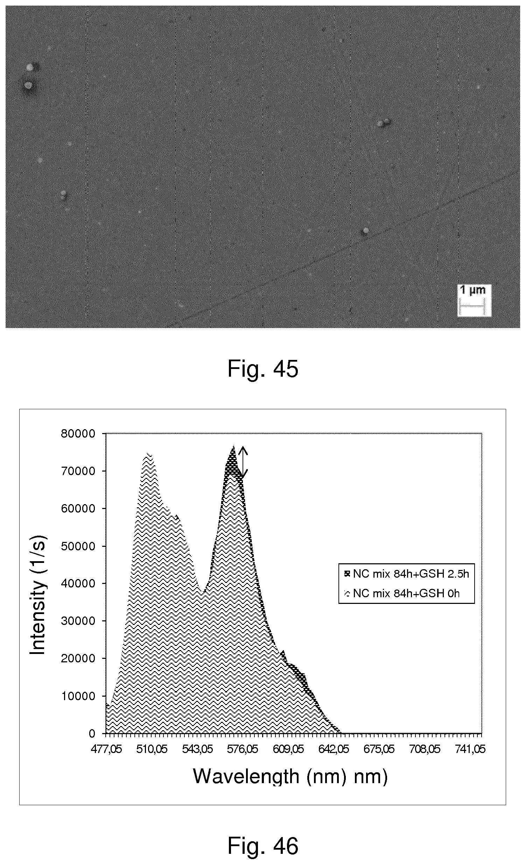

The surface of the titanium was observed using SEM and a fluorescence microscope in the case of nanocapsules containing fluorophore (See Fig. 45).

Cell adhesion and immunohistochemical tests were carried out to quantify the quality of the cell adhesion with satisfactory results.

This Example is a particularly advantageous embodiment of the invention.

### Antibacterial agents used

Roxithromycin, clarithromycin (hydrophobic antibacterial agents from the macrolide family). Via the functionalisation of titanium with nanocapsules that encapsulate this type of antibacterial agents, its biocompatibility increased for use in dental implants and for preventing bacterial adhesion to metal. These antibacterial agents can be encapsulated, for example, by means of the same encapsulating method as shown in Example 33.

### Fluorophore used

Clear blue DFSB-C0 (highly fluorescent hydrophobic molecule). It was encapsulated in microcapsules according to the invention and it was used to quantify in the fluorescence microscope the quality of the adhesion of nanocapsules to the titanium. The fluorophore can be encapsulated, for example, by means of the same Encapsulation Method as in Example 33.

### Adhesion promoting peptide used

The nanocapsules were covered with cRGDfK peptide following Example 32.

### Example 40. (AMPHIL-HYFOB) without cross-linking agent

This Example is the same as Example 38 but without adding a second compound, i.e.: without adding a cross-linker (L-lysine and DETA). Since no cross-linking agents are added, the nanocapsule is less stable in solution and degrades more easily when treated with GSH

In a reactor duly equipped with mechanical stirring at 50ºC and with an inert atmosphere 3.3814 g of IPDI, 0.75 g of Crodamol GTCC, 0.15 g of 2,2-dithiodiethanol (DEDS), 5.5 g of YMER N-120 and 10 mg of DBTL were added. When the theoretical NCO was reached (approximately after 4 to 5 hours reaction time) the heating bath was removed and 1.45 g of TAP 100D solubilised in 8 g of dry THF were added. The stirring was maintained and it was left to react for 30 min. This amphiphilic prepolymer is called AMPHIL in Figs. 38 and 40.

At the same time, in a 25 mL Schlenk at 50ºC purged with nitrogen, 0.486 g of IPDI, 0.15 g of DEDS and 10 mg of DBTL in 2 mL of dry THF were added. After 1 hour reaction time, 0.15 g of TAP 100D in 4 mL of dry THF was added and the heating was switched off. The stirring was maintained and it was left to react for 30 min. This hydrophobic prepolymer is called HYFOB in Figs. 39 and 40.

The reactor was cooled to about 5 to 10ºC and the hydrophobic prepolymer (HYFOB) and 0.904 g of Bayhydur 3100 in 16 ml of dry MEK was added on to the amphiphilic compound (AMPHIL). Bayhydur 3100 behaved as a linker (linking element) between the cRGDfK peptide and the nanocapsule (see Fig. 37). HYFOB was the hydrophobic prepolymer with disulphide bridges in the wall to make it degradable in the face of glutathione.

Then, dropwise, 140 mL de milliQ water basified to a pH between 11 and 12 cooled to a temperature between 5 and 10 ºC, was added.

After the inversion phase, 5 mL of triethylamine were added and left at room temperature for 72 hours until the NCO signal disappeared from the IR spectrum.

### Example 41

A experiment was conducted to know the release kinetics of the inside of the nanocapsules of a FRET pair (Forster resonance energy transfer) of hydrophobic fluorophores in an aqueous medium.

Example 38 was repeated and, on the one hand, a product was synthesised in which Dil (1,1'-dioctadecyl-3,3,3',3'-tetramethylindocarbocyanine perchlorate) was encapsulated and another product that encapsulates DiO (3,3'-dioctadecyloxacarbocyanine perchlorate). In both cases, for their encapsulation, Example 33 was followed. These two fluorophores represent a FRET pair, the DiO performing the function of donor and the Dil the acceptor. Once both products were synthesised, they were diluted to the desired concentration, mixed and the fluorescence analysis was carried out energising the donor to 450 nm.

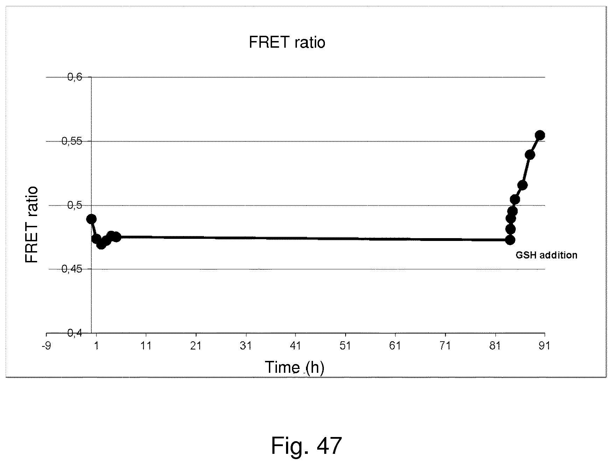

A mixture's fluorescence was followed-up for 84 hours at 37ºC without GSH. As can be seen in Figures 46 and 47, it was concluded that there was no exchange of fluorophores from inside the nanocapsules. This data proves that the nanocapsules were hermetic in an aqueous medium.

When a GSH solution was added to the mixture of nanocapsules, a progressive FRET increase was observed, since the wall of the nanocapsule degraded, allowing a flow of fluorophores from its inside to the outside, making them mix together and lead to a clear FRET increase. Figure 47 show the FRET ratio expressed as Iₐ/(I_{d} + Iₐ), where Iₐ and I_{d} are the fluorescence intensity of the acceptor (Dil) and donor (DiO), respectively.

This way, it is concluded that the nanocapsules are hermetic in an aqueous medium, but with GSH, they release hydrophobic fluorophores due to the degradation of the capsular wall as shown by FRET.

## Claims

1. Method for producing a microencapsulate comprising the following stages: [a] dispersing a first liquid phase in a second liquid phase forming an emulsion, so that said first phase remains dispersed in said second phase, and [b] forming a polymer which forms the wall of said microencapsulate, **characterised in that** between said first phase and said second phase an interphase is formed which comprises a reactive amphiphilic compound, where said amphiphilic compound is a prepolymer of said polymer, and said amphiphilic compound has at least two main functional groups that react in the subsequent polymerisation for forming said polymer, where said amphiphilic compound has at least one hydrophilic or hydrophobic functional group in a chain which is sideways with respect to the chain that links both main functional groups,
where said main functional groups of said amphiphilic compound are NCO functional groups, able to form urethane and/or urea type bonds, where said second phase comprises a second compound, where said second compound comprises at least two functional groups that react with said main functional groups of said amphiphilic compound, to form said polymer,
where said second compound is a polyamine, preferably a diamine, triamine, tetraamine or pentaamine and very preferably it is a polyamine from the group made up of ethylendiamine, diethylentriamine, triethylentetraamine and L-lysine,
where a second prepolymer is added to said first phase, where said second prepolymer has at least two main functional groups that react in the subsequent polymerisation for forming said polymer, where said second prepolymer is more hydrophobic than said amphiphilic compound if said first phase is organic, or said second prepolymer is more hydrophilic than said amphiphilic compound if said first phase is aqueous, so that said second prepolymer will tend to arrange itself as a second internal layer in the wall of the microcapsules, given its greater affinity to the first phase, and so that said second prepolymer will react also with the second compound thereby forming a wall with two layers, and
where said polymer comprises, in its main chain, a disulphide or an ester.

2. Method according to claim 1, **characterised in that** said two main functional groups of said amphiphilic compound are separated from each other by 4 to 12 bonds, preferably by 5 to 10 bonds.

3. Method according to one of claims 1 or 2, **characterised in that** a first precursor and a second precursor of said amphiphilic compound are added to said first liquid phase, and said first and second precursors are made to react with each other,
where said first precursor is an isocyanate from the group made up of IPDI, HDI and HMDI, and preferably it is IPDI,
where said second precursor is a hydrophilic compound, where said second precursor comprises at least two functional groups suitable for reacting with functional groups of said first precursor, said second precursor having its hydrophilic function in a chain which is sideways with respect to the chain that links the two functional groups that are suitable for reacting with functional groups of said first precursor,
where said second precursor is preferably a hydrophilic compound from the group made up of polyethoxylenated compounds with a molecular weight over 100, more preferably over 500, or said second precursor is preferably a diol or a diamine with a carboxylic function or sulphonic function in a chain which is sideways with respect to the chain that links the two alcoholic functional groups or amines, and more preferably it is DMPA (dimethylolpropionic acid).

4. Method according to claim 3, **characterised in that** a third precursor of said amphiphilic compound is added to said first liquid phase or to said second liquid phase, and it is made to react with the product of the reaction between said first and second precursors,
where said third precursor is a hydrophobic C₈-C₂₂ compound, where said third precursor comprises at least two functional groups suitable for reacting with functional groups of said first precursor, said third precursor having its hydrophobic function in a chain which is sideways with respect to the chain that links the two functional groups suitable for reacting with functional groups of said first precursor,
where said third precursor is preferably a compound from the group made up of fatty diols or diamines C₁₀-C₂₂, more preferably it is a glyceryl monoglyceride, a dimerdiol, a cocopropylendiamine or a C₁₆-C₁₈ aminopropylamine.

5. Method according to one of claims 3 or 4, **characterised in that** the two main functional groups of said first, second and/or third precursor are separated from one another by 4 to 12 bonds, preferably by 5 to 10 bonds.

6. Method according to any of the claims 3 to 5, **characterised in that** said first precursor has a degree of functionality higher than 3, and **in that** said second precursor and/or third precursor have a degree of functionality smaller than 2.

7. Method according to any of the claims 1 to 6, **characterised in that** said polymer comprises, in its main chain, a sulphonate, carboxylate or phosphate.

8. Method according to any of the claims 1 to 7, **characterised in that** said polymer or said amphiphilic compound comprises a functionalising group, preferably a peptide.

9. Method according to any of the claims 1 to 8, where said emulsion is an O/W emulsion, **characterised in that** it comprises a stage of adding at least one volatile, polar organic solvent to said first phase, before said stage [a], and a stage of evaporating said solvent, after said stage [b].

10. Microencapsulate obtained according to the method of any one of claims 1 to 9.

11. Microencapsulate according to claim 10, **characterised in that** it comprises, inside, an antitumoral active ingredient, preferably paclitaxel or plitidepsin, a photosensitive hydrophobic fluorophore, or an antibacterial agent, preferably a hydrophobic antibacterial agent, and very preferably a hydrophobic antibacterial agent from the macrolide family.

12. Cosmetic or pharmaceutical composition **characterised in that** it comprises a microencapsulate according to one of the claims 10 or 11.

13. Use of a microencapsulate according to one of the claims 10 or 11, for the superficial coating of a biocompatible material, preferably a biocompatible metal and very preferably titanium or its alloy.

## Patentansprüche

1. Verfahren zum Herstellen eines Mikrokapsulats, das die folgenden Schritte umfasst: [a] Dispergieren einer ersten Flüssigkeitsphase in einer zweiten Flüssigkeitsphase unter Bildung einer Emulsion, so dass die erste Phase in der zweiten Phase dispergiert bleibt, und [b] Bilden eines Polymers, das die Wand des Mikrokapsulats bildet, **gekennzeichnet dadurch, dass** zwischen der ersten Phase und der zweiten Phase eine Zwischenphase gebildet wird, die eine reaktive amphiphile Verbindung umfasst, wobei die amphiphile Verbindung ein Vorpolymer des Polymers ist, und wobei die amphiphile Verbindung mindestens zwei funktionelle Hauptgruppen aufweist, die in der darauffolgenden Polymerisierung zum Bilden des Polymers reagieren, wobei die amphiphile Verbindung mindestens eine hydrophile oder hydrophobe funktionelle Gruppe in einer Kette aufweist, die bezüglich der Kette, die die beiden funktionellen Hauptgruppen verbindet, seitwärts verläuft,
wobei die funktionellen Hauptgruppen der amphiphilen Verbindung funktionelle NCO-Gruppen sind, die Bindungen vom Urethan- und/oder Harnstofftyp bilden können,
wobei die zweite Phase eine zweite Verbindung umfasst, wobei die zweite Verbindung mindestens zwei funktionelle Gruppen umfasst, die mit den funktionellen Hauptgruppen der amphiphilen Verbindung reagieren, um das Polymer zu bilden,
wobei die zweite Verbindung ein Polyamin, vorzugsweise ein Diamin, Triamin, Tetraamin oder Pentaamin ist und besonders vorzugsweise ein Polyamin aus der Gruppe ist, die aus Ethylendiamin, Diethylentriamin, Triethylentetraamin und L-Lysin besteht,
wobei ein zweites Vorpolymer zu der ersten Phase hinzugefügt wird, wobei das zweite Vorpolymer mindestens zwei funktionelle Hauptgruppen aufweist, die in der darauffolgenden Polymerisierung zum Bilden des Polymers reagieren, wobei das zweite Vorpolymer hydrophober ist als die amphiphile Verbindung, falls die erste Phase organisch ist, oder das zweite Vorpolymer hydrophiler ist als die amphiphile Verbindung, falls die erste Phase wässrig ist, so dass das zweite Vorpolymer dazu neigen wird, sich als eine zweite innere Schicht in der Wand der Mikrokapseln aufgrund seiner größeren Affinität zur ersten Phase anzuordnen, und so dass das zweite Vorpolymer auch mit der zweiten Verbindung reagieren wird, um dadurch eine Wand mit zwei Schichten zu bilden, und
wobei das Polymer in seiner Hauptkette ein Disulfid oder einen Ester umfasst.

2. Verfahren nach Anspruch 1, **gekennzeichnet dadurch, dass** die zwei funktionellen Hauptgruppen der amphiphilen Verbindung voneinander durch 4 bis 12 Bindungen, vorzugsweise durch 5 bis 10 Bindungen, getrennt sind.

3. Verfahren nach einem der Ansprüche 1 oder 2, **gekennzeichnet dadurch, dass** ein erster Vorläufer und ein zweiter Vorläufer der amphiphilen Verbindung zu der ersten Flüssigkeitsphase hinzugefügt werden und dass der erste und der zweite Vorgänger miteinander reagieren gelassen werden,
wobei der erste Vorläufer ein Isocyanat aus der Gruppe ist, die aus IPDI, HDI und HMDI zusammengesetzt ist, und vorzugsweise IPDI ist,
wobei der zweite Vorläufer eine hydrophile Verbindung ist, wobei der zweite Vorläufer mindestens zwei funktionelle Gruppen umfasst, die sich zur Reaktion mit den funktionellen Gruppen des ersten Vorläufers eignen, wobei der zweite Vorläufer seine hydrophile Funktion in einer Kette aufweist, die bezüglich der Kette, die die zwei funktionellen Gruppen verbindet, die sich zur Reaktion mit funktionellen Gruppen des ersten Vorläufers eignen, seitwärts verläuft,
wobei der zweite Vorläufer vorzugsweise eine hydrophile Verbindung aus der Gruppe ist, die aus polyethoxylenierten Verbindungen mit einem Molekulargewicht über 100, besonders vorzugsweise über 500, besteht, oder der zweite Vorläufer vorzugsweise ein Diol oder ein Diamin mit einer Karbonfunktion oder Sulphonfunktion in einer Kette ist, die bezüglich der Kette, die die zwei alkoholischen funktionellen Gruppen oder Amine verbindet, seitwärts verläuft, und besonders vorzugsweise DMPA (Dimethylolpropionsäure) ist.

4. Verfahren nach Anspruch 3, **gekennzeichnet dadurch, dass** ein dritter Vorläufer der amphiphilen Verbindung zu der ersten Flüssigkeitsphase oder zu der zweiten Flüssigkeit hinzugefügt wird und mit dem Produkt der Reaktion zwischen dem ersten und dem zweiten Vorläufer reagieren gelassen wird,
wobei der dritte Vorläufer eine hydrophobe C₈-C₂₂-Verbindung ist, wobei der dritte Vorläufer mindestens zwei funktionelle Gruppen umfasst, die sich zur Reaktion mit funktionellen Gruppen des ersten Vorläufers eignen, wobei der dritte Vorläufer seine hydrophobe Funktion in einer Kette aufweist, die bezüglich der Kette, die die zwei funktionellen Gruppen verbindet, die sich zur Reaktion mit funktionellen Gruppen des ersten Vorläufers eignen, seitwärts verläuft,
wobei der dritte Vorläufer vorzugsweise eine Verbindung aus der Gruppe ist, die aus Fettdiolen oder Diaminen C₁₀-C₂₂ besteht, besonders vorzugsweise ein Glycerylmonoglycerid, ein Dimerdiol, ein Kokopropylendiamin oder ein C₁₆-C₁₈ Aminopropylamin ist.

5. Verfahren nach einem der Ansprüche 3 oder 4, **gekennzeichnet dadurch, dass** die zwei funktionellen Hauptgruppen des ersten, des zweiten und/oder des dritten Vorläufers voneinander durch 4 bis 12 Bindungen, vorzugsweise durch 5 bis 10 Bindungen, getrennt sind.

6. Verfahren nach einem der Ansprüche 3 bis 5, **gekennzeichnet dadurch, dass** der erste Vorläufer einen Funktionalitätsgrad höher als 3 aufweist und dass der zweite Vorläufer und/oder der dritte Vorläufer einen Funktionalitätsgrad von kleiner als 2 aufweisen.

7. Verfahren nach einem der Ansprüche 1 bis 6, **gekennzeichnet dadurch, dass** das Polymer in seiner Hauptkette ein Sulfonat, Carboxylat oder Phosphat umfasst.

8. Verfahren nach einem der Ansprüche 1 bis 7, **gekennzeichnet dadurch, dass** das Polymer oder die amphiphile Verbindung eine Funktionalisierungsgruppe, vorzugsweise ein Peptid umfasst.

9. Verfahren nach einem der Ansprüche 1 bis 8, wobei die Emulsion eine O/W-Emulsion ist, **gekennzeichnet dadurch, dass** es einen Schritt des Hinzufügens mindestens eines volatilen, polaren organischen Lösungsmittels zur ersten Phase vor dem Schritt [a] und einen Schritt des Ausdampfens des Lösungsmittels nach dem Schritt [b] umfasst.

10. Mikrokapsulat, das nach dem Verfahren nach einem der Ansprüche 1 bis 9 erhalten worden ist.

11. Mikrokapsulat nach Anspruch 10, **gekennzeichnet dadurch, dass** es darin einen antitumoralen Wirkstoffbestandteil, vorzugsweise Paklitaxel oder Plitidepsin, einen fotoempfindlichen hydrophoben Fluorophor oder einen antibakteriellen Wirkstoff, vorzugsweise einen hydrophoben antibakteriellen Wirkstoff und besonders vorzugsweise einen hydrophoben antibakteriellen Wirkstoff aus der Makrolidfamilie umfasst.

12. Kosmetische oder pharmazeutische Zusammensetzung, **gekennzeichnet dadurch, dass** sie ein Mikrokapsulat nach einem der Ansprüche 10 oder 11 umfasst.

13. Verwendung eines Mikrokapsulat nach einem der Ansprüche 10 oder 11 für die Oberflächenbeschichtung eines biokompatiblen Materials, vorzugsweise eines biokompatiblen Metalls und besonders vorzugsweise von Titanium oder einer Legierung davon.

## Revendications

1. Procédé permettant de produire un microencapsulé, comprenant les étapes suivantes : [a] dispersion d'une première phase liquide dans une seconde phase liquide formant une émulsion, de sorte que ladite première phase reste dispersée dans ladite seconde phase, et [b] formation d'un polymère qui forme la paroi dudit microencapsulé, **caractérisé en ce qu'**entre ladite première phase et ladite seconde phase est formée une interphase qui comprend un composé amphiphile réactif, où ledit composé amphiphile est un prépolymère dudit polymère, et ledit composé amphiphile a au moins deux groupes fonctionnels principaux qui réagissent lors de la polymérisation consécutive pour former ledit polymère, où ledit composé amphiphile a au moins un groupe fonctionnel hydrophile ou hydrophobe dans une chaîne qui est latérale par rapport à la chaîne qui relie les deux groupes fonctionnels principaux,
où lesdits groupes fonctionnels principaux dudit composé amphiphile sont des groupes fonctionnels NCO, aptes à former des liaisons de type uréthane et/ou urée,
où ladite seconde phase comprend un second composé, où ledit second composé comprend au moins deux groupes fonctionnels qui réagissent avec lesdits groupes fonctionnels principaux dudit composé amphiphile pour former ledit polymère,
où ledit second composé est une polyamine, de préférence une diamine, une triamine, une tétra-amine ou une penta-amine et est très préférablement une polyamine issue du groupe constitué par l'éthylènediamine, la diéthylènetriamine, la triéthylènetétra-amine et la L-lysine,
où un deuxième prépolymère est ajouté à ladite première phase, où ledit deuxième prépolymère a au moins deux groupes fonctionnels qui réagissent lors de la polymérisation consécutive pour former ledit polymère, où ledit deuxième prépolymère est plus hydrophobe que ledit composé amphiphile si ladite première phase est organique, ou ledit deuxième prépolymère est plus hydrophile que ledit composé amphiphile si ladite première phase est aqueuse, de sorte que ledit deuxième prépolymère aura tendance à s'agencer en tant que seconde couche interne dans la paroi des microcapsules, étant donnée sa plus grande affinité avec la première phase, et de sorte que ledit deuxième prépolymère réagira également avec le second composé, formant ainsi une paroi avec deux couches, et
où ledit polymère comprend, dans sa chaîne principale, un disulfure ou un ester.

2. Procédé selon la revendication 1, **caractérisé en ce que** lesdits deux groupes fonctionnels principaux dudit composé amphiphile sont séparés l'un de l'autre par 4 à 12 liaisons, de préférence par 5 à 10 liaisons.

3. Procédé selon l'une des revendications 1 ou 2, **caractérisé en ce qu'**un premier précurseur et un deuxième précurseur dudit composé amphiphile sont ajoutés à ladite première phase liquide, et lesdits premier et deuxième précurseurs sont produits pour réagir l'un avec l'autre,
où ledit premier précurseur est un isocyanate issu du groupe constitué par l'IPDI, l'HDI et l'HMDI, et est de préférence l'IPDI,
où ledit deuxième précurseur est un composé hydrophile, où ledit deuxième précurseur comprend au moins deux groupes fonctionnels appropriés pour réagir avec des groupes fonctionnels dudit premier précurseur, ledit deuxième précurseur ayant sa fonction hydrophile dans une chaîne qui est latérale par rapport à la chaîne qui relie les deux groupes fonctionnels qui sont appropriés pour réagir avec des groupes fonctionnels dudit premier précurseur,
où ledit deuxième précurseur est de préférence un composé hydrophile issu du groupe constitué par des composés polyéthoxylénés ayant un poids moléculaire de plus de 100, plus préférablement de plus de 500, ou ledit deuxième précurseur est de préférence un diol ou une diamine ayant une fonction carboxylique ou une fonction sulfonique dans une chaîne qui est latérale par rapport à la chaîne qui relie les deux groupes fonctionnels alcooliques ou les amines, et est plus préférablement le DPMA (acide diméthylolpropionique).

4. Procédé selon la revendication 3, **caractérisé en ce qu'**un troisième précurseur dudit composé amphiphile est ajouté à ladite première phase liquide ou à ladite seconde phase liquide, et **en ce qu'**il est formé pour réagir avec le produit de la réaction entre lesdits premier et deuxième précurseurs,
où ledit troisième précurseur est un composé hydrophobe en C₈-C₂₂, où ledit troisième précurseur comprend au moins deux groupes fonctionnels appropriés pour réagir avec des groupes fonctionnels dudit premier précurseur, ledit troisième précurseur ayant sa fonction hydrophobe dans une chaîne qui est latérale par rapport à la chaîne qui relie les deux groupes fonctionnels appropriés pour réagir avec des groupes fonctionnels dudit premier précurseur,
où ledit troisième précurseur est de préférence un composé issu du groupe constitué par des diols gras ou des diamines en C₁₀-C₂₂, et est plus préférablement un monoglycéride de glycéryle, un dimère-diol, une copropylènediamine ou une aminopropylamine en C₁₆-C₁₈.

5. Procédé selon l'une des revendications 3 ou 4, **caractérisé en ce que** les deux groupes fonctionnels principaux desdits premier, deuxième et/ou troisième précurseurs sont séparés l'un de l'autre par 4 à 12 liaisons, de préférence par 5 à 10 liaisons.

6. Procédé selon l'une quelconque des revendications 3 à 5, **caractérisé en ce que** ledit premier précurseur a un degré de fonctionnalité supérieur à 3, et **en ce que** ledit deuxième précurseur et/ou ledit troisième précurseur a/ont un degré de fonctionnalité inférieur à 2.

7. Procédé selon l'une quelconque des revendications 1 à 6, **caractérisé en ce que** ledit polymère comprend, dans sa chaîne principale, un sulfonate, un carboxylate ou un phosphate.

8. Procédé selon l'une quelconque des revendications 1 à 7, **caractérisé en ce que** ledit polymère ou ledit composé amphiphile comprend un groupe fonctionnalisant, de préférence un peptide.

9. Procédé selon l'une quelconque des revendications 1 à 8, dans lequel ladite émulsion est une émulsion H/E, **caractérisé en ce qu'**il comprend une étape d'ajout d'au moins un solvant organique polaire volatil à ladite première phase, avant ladite étape [a], et une étape d'évaporation dudit solvant après ladite étape [b].

10. Microencapsulé obtenu selon le procédé de l'une quelconque des revendications 1 à 9.

11. Microencapsulé selon la revendication 10, **caractérisé en ce qu'**il comprend, à l'intérieur, un principe actif antinéoplasique, de préférence le paclitaxel ou la plitidepsine, un fluorophore hydrophobe photosensible, ou un agent antibactérien, de préférence un agent antibactérien hydrophobe, et très préférablement un agent antibactérien hydrophobe issu de la famille des macrolides.

12. Composition cosmétique ou pharmaceutique **caractérisée en ce qu'**elle comprend un microencapsulé selon l'une des revendications 10 ou 11.

13. Utilisation d'un microencapsulé selon l'une des revendications 10 ou 11 pour l'enrobage superficiel d'un matériau biocompatible, de préférence un métal biocompatible, et très préférablement le titane ou son alliage.
